(19) Europäisches Patentamt
European Patent Office
Office européen des brevets

(11) EP 0 481 930 B1

(12) **EUROPEAN PATENT SPECIFICATION**

(45) Date of publication and mention of the grant of the patent:
**18.06.1997 Bulletin 1997/25**

(51) Int Cl.⁶: **C07K 7/02**, C07K 14/00, C07K 14/525, C07K 14/575, G01N 33/68

(21) Application number: **91830428.8**

(22) Date of filing: **14.10.1991**

(54) **Nonlinear peptides hydropathycally complementary to known amino acid sequences, process for the production and uses thereof**

Nicht-lineare Peptide, die hydropatisch komplementär zu bekannten Aminosäuresequenzen sind, Verfahren zur Herstellung und Verwendung davon

Peptides non-linéaires complémentaires hydropathiquement à des séquences connues d'acides aminés, leur préparation et leurs applications

(84) Designated Contracting States:
**AT BE CH DE DK ES FR GB GR IT LI LU NL SE**

(30) Priority: **15.10.1990 IT 4836590**
**15.04.1991 IT RM910261**

(43) Date of publication of application:
**22.04.1992 Bulletin 1992/17**

(60) Divisional application: **96114931.7**

(73) Proprietor: **TECNOGEN S.C.P.A.**
**81015 Piana de Monte Verna (Caserta) (IT)**

(72) Inventors:
- **Fassina, Giorgio,**
**c/o TECNOGEN Società Consortile**
**I-20131 Milano (IT)**
- **Corti, Angelo,**
**c/o TECNOGEN Società Consortile**
**I-20131 Milano (IT)**

(74) Representative: **de Simone, Domenico et al**
**Ing. Barzanò & Zanardo Roma S.p.A.**
**Via Piemonte 26**
**00187 Roma (IT)**

(56) References cited:
**WO-A-90/11573**

- **PROCEEDINGS OF THE NATIONAL ACADEMY OF SCIENCES , USA vol. 85, August 1988, WASHINGTON, USA pages 5409 - 5413 TAM, J.P. 'Synthetic peptide vaccine design: synthesis and properties of a high-density multiple antigenic peptide system'**
- **BIOCHEMISTRY vol. 28, no. 22, 1989, EASTON, USA pages 8804 - 8811 SHAI ET AL**
- **BIOCHEMISTRY vol. 28, no. 22, 1989, EASTON, USA pages 8811 - 8818 FASSINA ET AL 'Recognition properties of antisense peptides to Arg- 8 - vasopressi / bovine neurophysin II biosynthetic precursor sequences'**
- **EDITORS: RIVIER ET AL // AUTHORS: AL-OBEIDI ET AL 'Proceedings of the 11th American Peptide Symposium 1989 // Antisense peptides of melanocyte- stimulating hormone (MSH): Surprising results' 1990 , ESCOM , LEIDEN, HOLLAND**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 481 930 B1

## Description

This invention relates to nonlinear peptides hydropathically complementary to known amino acid sequences, and to a process for the production and uses thereof.

More particularly, this invention relates to two or more peptides hydropathically complementary to known amino acid sequences, said peptides being linked non-linearly to an amino acid nucleus comprising at least one amino acid having two terminal amino functions, and the invention also relates to the process for synthesizing said peptides, as well as to the immobilization of such molecules on solid supports to be employed for in vitro diagnostic determinations, for affinity chromatography separations, for analytical procedures for the determination of peptide analytes in complex matrices, for identifying reaction activities with said peptides, more particularly proteolytic activities, for identifying activities which inhibit said proteolytic activities, in biological fluids, fermentation broths, conditioned culture soils, cell extracts, plant extracts.

The hypothesis that interaction sites between pairs of interacting proteins are coded by complementary DNA sequences has been first formulated by Biro (Biro, Medical Hypothesis 7:669, 1981 ) and then confirmed experimentally by Blalock et al. (Bost et al., PNAS 82: 1372, 1985). The latters have shown a meaningful hydropathic complementarity between amino acids decuced from complementary codons (Blalock and Smith, BBRC, 121:203, 1984), read in the 3'-5'or the 5'-3'directions (Elalock and Bost, Biochem. J., 234:679, 1986). The initial observations have then been confirmed experimentally in a large number of other systems of biological importance, both allowing the ex novo design to be carried out of peptides capable of associating target peptides or proteins, as is the case of fibronectin (Brentani et al., PNAS, 85:364, 1988), insulin (Knutson, J. Biol. Chem., 263:14146, 1988), Arg8-vasopressing (Fassina et al., Biochemistry, 28:8811, 1989), the substance P (Bost and Blalock, Methods in Enzymology, 168:16, 1989), ribonuclease peptide S (Shai et al., Biochemistry, 28:8804, 1989), the C-raf protein (Fassina et al., J. Biol. Chem., 264:11252, 1989), and allowing the prediction of the contact sites between proteins and their receptors to be performed as well, like in the case of interleukin 2, of transferring and of the EGF or Epidermal Growth Factor (Bost et al., BBRC 28:1375, 1985).

Recently the authors of this invention have realized (Italian Patent Application No. 21396 A/89 and No. 20755 A/90) a computer-aided procedure that allows peptide chains hydropathically complementary to be obtained capable of interacting with given amino acid sequences, employing just primary sequence information of said sequences. The peptides so obtained show to have a higher binding affinity with respect to those obtained from complementary DNA, and they can be employed advantageously in diagnostic and analytical procedures, which are often carried out in the solid phase.

The methods already known for the immobilization of low molecular weight peptides (M.W. 1000-4000) on solid supports can be essentially divided into the methods that are based on a covalent interaction between the peptide and the support, and the methods in which the peptide is linked or adsorbed in a non-covalent way. In the first instance, generally the chemical reactivity of the amino or carboxyl groups of the peptides is exploited for esterification reactions with suitable groups which are present on the solid support. As quite often the peptide of interest can contain more than one reactive group (for instance, amino or carboxyl groups), the immobilization can be hardly selected towards a particular group, so being non-homogeneous and unforeseeable. In the case in which the peptide has a single functional group, the immobilization turns out to be oriented, but the closeness to the solid phase quite often causes a partial or total loss of the initial recognition capabilities. The introduction of suitable spacers between the solid phase and the peptide obviates some drawbacks of such kind, but such approach considerably increases the cost of the support or of the peptide, considerably decreases the total capability, and in addition it is to be adjusted in its details for each peptide individually, and introduces some changes which are due to non-specific interactions between the spacer and the other molecules. The immobilization of peptides by means of non-specific interactions on solid supports is also illustrated in the literature (Geerligs et al., J. Immunol. Methods, 106: 239, 1988). In that case, the orientation of the peptide is fully undeterminable, because many residues are involved in the interaction in an unforeseeable way.

Moreover, the same authors have found a remarkable limitation to the employment of hydropathically complementary peptides which are the object of the Italian Patent Application No. 20755 A/90, because of the loss or reduction of affinity when, employing the methods disclosed, they are immobilized on a solid phase,being it a solid support previously derivatized for preparing as affinity chromatographic column, or a plastic plate which has been suitably adjusted to carry out analytical tests.

Recently (Tam, Proc. Natl. Acad. Sci. USA 85:5409, 1988) a procedure has been realized for the production of antipeptide antibodies employing multiple peptides as antigens, said peptides being covalently linked to an octadentate polylysine nucleus. The resulting molecules shows an increased immunogenic activity, up to a degree much higher than that of the same linear peptides.

The authors of the present invention have surprisingly found that the covalent or non-covalent immobilization on solid supports of peptides which are hydropathically complementary to known peptides, and are synthesized in a multiple and non linear form according to a process similar to Tam's procedure (cf. above) for the production of antigenic multiple peptides, allows many of the drawbacks described previously to be obviated. Indeed, during the immobilization

process, just some of the peptide chains of the multimer molecule are statistically involved in the interaction with the solid phase, whereas the other ones show fully accessible and properly oriented to carry out the recognition with hydropathically complementary peptide sequences. Accordingly, it is possible to employ the affinity of hydropathically complementary peptides in diagnostic tests and in analytical and preparative methodologies which make use of affinity chromatographic partitions and, in general, in the field of immobilization of the peptide ligands. In particular, hydropathically complementary peptides can be employed for identifying reacting activities with the same. Indeed, in the industrial pharmaceutical field, the search for new, pharmacologically active molecules is carried out through the study of the biological activities of fermentation broth, of conditioned culture soils, of cell extracts, of plant extracts and of other products of fermentation origin containing a large number of bioorganic molecules which are uncharacterized and are endowed with activities of the most different kinds.

The peptides and the proteins, which comprise also those having hormonal actions, endowed with pharmacological activities and which, while biologically synthesized originally in the form of high molecular weight precursors, require activation by means of sequence-specific proteolytic enzymes, are numerous. Accordingly, in the pharmacological field the research of both such enzymes and of molecules capable of inhibiting such conversions for contrasting the effect of the same is very important.

Generally, the search for enzymes and consequently for their specific inhibitors is made easier by the availability of chromogenic synthetic substrates that, mimicking the natural substrate, allow the enzymatic activity of biological extracts not necessarily purified to be monitored, as well as the effect of molecules having a possible inhibiting activity. However, in many cases, said synthetic substrates are not available and the enzymatic conversion reactions are to be followed directly on the natural substrate, with remarkable problems connected quite often to the low detection limits obtainable and to the complexity of the tests which can be employed. Anyway, even in the case of chromogenic substrates, the tests for detecting proteolytic activities or the presence of inhibitors show to be hardly adaptable to the rapid, convenient and sensitive procedures in the solid phase, which are increasingly employed in the diagnostic field, but up to the present time are not exploited for tests on protease activities or on activities which inhibit the same.

Recently a 21-amino acid peptide has been identified and characterized, which has been called "endothelin" (El) and is endowed with a powerful vasoconstricting action (Yanagisawa et al., 1988, Nature 332: 411-415). Endothelin is synthesized by the endothelial cells in the form of a precursor having 200 amino acids (preproendothelin), first converted into pro-endothelin (38 amino acids) and then into mature endothelin (1-21) through proteolytic degradation by means of one or more specific enzymes which have not been characterized as yet and are called "ECE" (endothelin converting enzymes). Pro-endothelin is pharmacologically inactive, but it can be activated both in vitro and in vivo, also by the action of wide-spectrum proteolytic enzymes like chymotrypsin and cathepsin. Moreover, it has also been shown that the presence of protease inhibitors like phosphoramidone not only prevent pro-endothelin from being converted into endothelin, but also prevents the blood pressure from increasing in rats when proendothelin is administered to the same through injection. It is of remarkable importance that such inhibitor gradually reduces also the blood pressure in rats suffering from spontaneous hyper-tension (McMahon et al., 1990, Proc. 2nd Internat. Meeting on Endothelin, Tsukuba City, Japan).

ECE chromogenic substrates for employment in the identification of such proteolytic activities or of similar proteolytic activities as well as of inhibitors of said activities are not available at present.

Accordingly, it is clearly evident there is the need for a rapid, sensitive, automatizable process that can be easily carried out for the identification and characterization of proteolytic activities or of activities capable of inhibiting the same, in biological fluids, fermentation broth, conditioned culture soils, cell extracts, plant extracts, which process, in particular, can be employed for the research of ECE inhibitors more specific and more active than those already available, for obtaining new molecules having hypotensive activity, as well as for the isolation and the characterization of ECE itself, in order to design and synthesize in vitro synthetic molecules having an inhibiting activity of the proteolytic activities.

According to this invention, the authors of the same have surprisingly found that also the pro-endothelin 16-32 fragment undergoes a proteolytic degradation at the level of the 21-22 residues, because of the action of enzymes like chymotrypsin, the kinetic characteristics being similar to those observed with the whole pro-endotlelin. The 8ΔET (16-29) molecule, both in solution and in the solid phase, recognizes the fragment 16-32 of pro-endothelin, even though it has been derivatized with biotin, whereas it is uncapable of associating the fragments 16-21 and/or 22-32 obtained by chymotryptic degradation. Accordingly, it has been possible to exploit the immobilization of the 8ΔET (16-29) molecule on plastic plates in order to design a solid-phase test for detecting the derivatized biotin ET (16-32). Such test allows many of the drawbacks previously met with in the search for proteolytic enzymes to be obviated, in particular with specific activities towards pro-ET and of activities inhibiting the same.

Accordingly, the object of the present invention consists in nonlinear peptides which are hydropathically complementary to proteins, peptides or portions of the same, at least a portion of the amino acid sequence of which is known, that comprise:

an amino acid nucleus with at least one amino acid having at least two terminal amino functions and a linear

amino acid sequence hydropathically complementary to the portion of the known amino acid sequence, which is linked through a carboxy amino link to each one of said terminal amino functions.

The amino acid nucleus according to the present invention has the following formula:

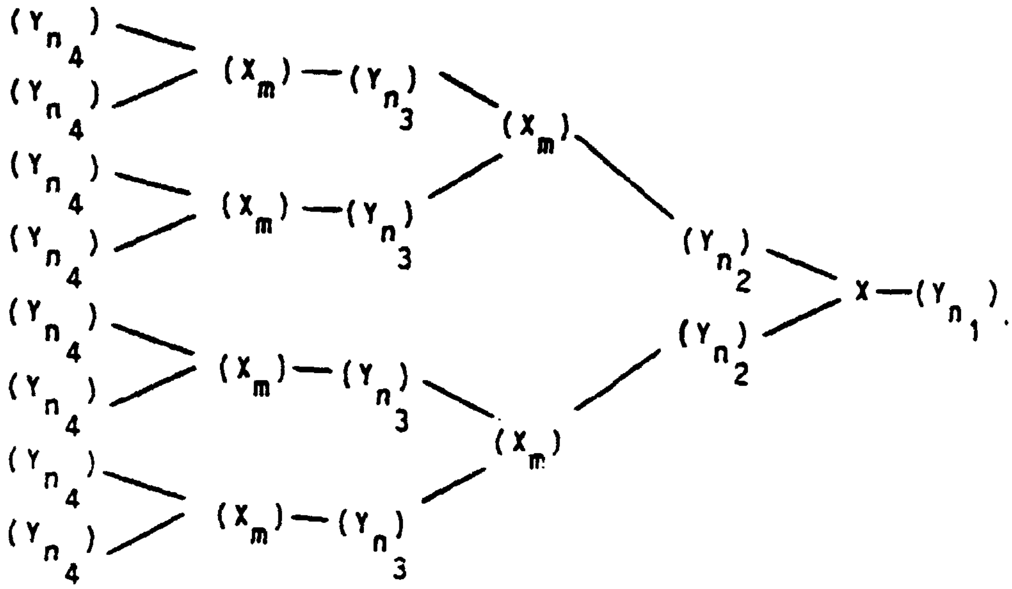

wherein X is an amino acid with at least two amino functional residues, Y is an amino acid which is selected from the group made up of alanine, glycine or arginine; m is a number between 0 and 1; $n_1$, $n_2$, $n_3$ and $n_4$ are numbers in the range from 0 and 10, and wherein the bonds are carbamido bonds.

Preferably the linear, hydropathically complementary amino acid portions are made up of a number of amino acids between 10 and 20.

According to a preferred embodiment of this invention, the amino acids having at least two terminal amino functions are selected between lysine and ornitine.

The employment of molecules which are the object of this invention in solid-phase partition procedures avoids the employment of spacers introduced synthetically in order to cause low molecular weight peptides to be more accessible to the interaction with high molecular weight protein macromolecules. Indeed, the chains which are linked directly to the solid support act like internal spacers, as they are made up of a linear polyatomic series of 3n atoms, wherein n represents the number of amino acid residues that makes part of the peptide unit of the multimeric compound. Moreover, non-specific interactions which are often associated to the employment of 6-12 carbon atoms aliphatic spacers are not introduced.

Again according to the present invention, the multimeric peptide is directly immobilized with no previous manipulations on pre-activated supports which are commercially available, so obviating the need for difficult and costly immobilization procedures which often give low yields of the final product.

This invention will be disclosed now in the aspect that relates to the synthesis of hydropathically complementary peptides (pointed out with the simbol Δ) to portions of amino acid sequence made of two proteins: the "Big Endothelin" (Big ET, Itoh et al., Febs. Lett., 231, 440) from the amino acid 16 to the amino acid 29, and the "Tumor Necrosis Factor" (TNFα , Beutler and Cerami, Ann. Rev. Immunolog. 7, 625, 1989) from the amino acid 144 to the amino acid 157, covalently linked in a nonlinear manner to a lysine nucleus so as to form an octadentate molecule; to their employment for the preparation of affinity chromatographic columns and to the relative purification of the Big ET peptide as well as of the protein TNFα ; to the immobilization of said molecules on a solid support for the quantitative determination of Big ET and TNFα .

It is a further object of this invention a process for the identification of proteolytic activities and/or of inhibitors of such activities, said process comprising the reaction

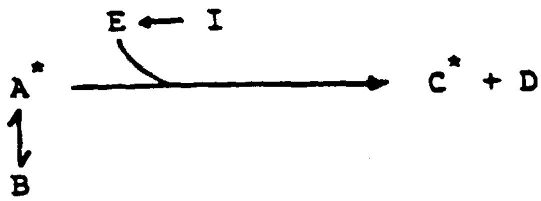


wherein

E represents said proteolytic activity, capable of catalyzing the conversion of a substrate A comprising at least one protein fragment labelled with at least one site of proteolytic attack, to the fragments C and D,
I represents said inhibitors of such conversion, B represents a ligand which is capable of recognizing and bonding said substrate A but not said fragments C and D; B consisting of molecules made up of an amino acid nucleus comprising at least one amino acid having at least two terminal amino functions and a linear amino acid sequence which is hydropathically complementary to said protein fragment and is linked, through a carbamido link, to each one of said terminal amino functions and the asterisk points out that said substrate A is labelled.

According to the present invention, said fragment is labelled by means of radioisotopes, or of enzymes, or of fluorescent compounds or of chromophores, or of biotin.

Preferably, said fragment is labelled with biotin and can be evidenced by incubation with streptavidin conjugated with peroxidase.

Again according to this invention, the process comprising means for the separation of the protein fragment-ligand complex from unlinked protein fragments and from the proteolysed derivatives.

Said separation means are conveniently realized through the immobilization on a solid phase of said ligand, and washing off said protein fragments which are not bonded and said proteolysed derivatives.

Again according to the present invention, said solid phase comprises plastic plates divided into compartments.

Again according to this invention, the process comprises the step of measuring said labelled protein fragments linked to said ligand immobilized on a solid phase.

Again it is an object of the present invention a process wherein said protein substrate comprises a fragment of the pro-endothelin and said ligand comprises amino acid sequences which are idropathically complementary to said fragment of the pro-endothelin.

Again more preferably, said fragment of the pro-endothelin is made up of the amino acid 16 up to the amino acid 32 of the formula HLDIIWVNTPEHIVPYG, and said detection molecule comprises the molecule 8ΔET (16-29).

According to the present invention, said proteolytic attack site is comprised between the amino acids 21-22 of the fragment 16-32 of pro-endothelin, in such a way as to give rise, if in the presence of proteolytic activities, to two fragments ET (16-21) and ET (22-32) which do not become linked to said molecule 8ΔET (16-29).

The presence of proteolytic activities suitable to convert said substrate ET (16-32) to ET (16-21) and to ET (22-32) is easily put into evidence, through the incubation of suitable amounts of said biotin-derivatized substrate, with the sample under test, and determining the amount of biotinized derivative linked to the plates. In a similar way, the presence of inhibitors of the conversion reaction is put into evidence through the incubation of suitable amounts of biotinized derivative and chymotrypsin solutions or raw preparations of ECE, with the sample under test, and then determining the amount of said biotinized substrate linked to the plate.

This invention will be disclosed in the following just for illustrative and not for limitative purposes by means of the following application examples, wherein the following figures will be referred to, wherein:

Figure 1A represents the structure of the polylysine nucleus employed for the synthesis of 8ΔET (16-29);
Figure 1B represents the structure of the nonlinear peptide 8ΔET (16-29);
Figure 1C represents the structure of the peptide ET (16-29);
Figure 1D represents the structure of the nonlinear peptide 8ET (16-29);
Figure 2 illustrates the different performance of the columns prepared with ΔET (16-29) and 8ΔET (16-29);
Figure 3A illustrates the purification of the biotinylated derivative of ET (1-32) on an affinity column prepared with the peptide 8ΔET (16-29);
Figure 3B illustrates an analysis carried out by means of RP-HPLC of the biotin ET (1-32) product purified through affinity chromatography;
Figure 4 illustrates the binding of the biotinylated derivative of ET (1-32) with plastic microcontainers derivatized

with 8ΔET (16-29);

Figure 5 illustrates the interaction specificity between ET (1-32) and 8ΔET (16-29) immobilized on plastic microcontainers;

Figure 6 illustrates the various affinity of the peptides Et (16-29) (A) and 8ET (16-29) (B) towards 8ΔEt (16-29);

Figure 7A illustrates the structure of the polylysine nucleus employed for the synthesis of 8ΔTNF (144-157);

Figure 7B illustrates the structure of the peptide 8ΔTNF (144-157);

Figure 8A represents the binding of TNFα with an affinity column prepared with the peptide 8ΔTNF (144-157);

Figure 8B represents an analysis carried out through RP-HPLC of the TNFα purified by means of affinity chromatography; and

Figure 9 illustrates the specificity of interaction between TNFα and 8ΔTNF (144-157) immobilized on plastic microcontainers;

Figure 10 represents the kinetic curves of degradation of pro-endothelin and pro-endothelin 16-32 by the action of enzyme alpha-chymotrypsin;

Figure 11 represents the binding of the biotinized derivative of ET (16-32) with plates sensitized with 8ΔET (16-29) in the presence and in the absence of alpha-chymotrypsin; and

Figure 12 illustrates the bond of the biotinylated derivative of ET (16-32) with plates sensitized with 8ΔET (16-29) previously treated with alpha-chymotrypsin both in the presence and in the absence of inhibitors.

This invention has been employed for synthesizing sequences of nonlinear peptides which are hydropathically complementary to the site 16-29 of the Big Endothelin (Yanagisawa et al., Nature 322, 411 (1988)) [1-38], and to the site 144-157 of the TNFα(Bentler and Cerami, Ann. Rev. Immunol., 7:625, 1989), so obtaining a better exploitation of the same for the immobilization on solid phases. The peptides can be synthesized on the basis of procedures which are already known to those who are skilled in the art, in solution or through solid-phase synthesis, starting from a polylysine nucleus formed by the covalent union of seven lysine residues as illustrated in Figure 1A. The peptide 8ΔET [16-29], whose structure is shown in Figure 1B, has been synthesized by solid-phase synthesis following a Fmoc procedure (Dryland and Sheppard, TH 44, 859 (1988)) employing as the solvent system N-methylpyrrolidone/dichloromethane, employing an automatic synthesizer Applied Biosystem 431A, and following the hints of the maker. Similarly, the nonlinear peptide 8ΔTNF [144-157] has been synthesized, whose sequence is shown in Figure 7B. The synthesis and purification procedures for the peptides are largely represented and illustrated in the literature, and such procedures are well known to those who are skilled in the art. The peptide 8ΔET [16-29] has been employed for preparing an affinity column for the purification of Big Et from mixtures containing 20 different peptides, each one at a concentration of 1 mg/ ml. As is shown in the following examples, affinity columns prepared with the 8ΔET [16-29] peptide show a higher binding capacity, i.e. they are capable of purifying higher amounts of Big ET with respect to columns prepared through the immobilization of the peptide ΔET [16-29] (RKFLAGLRARRLKF) in equimolar amounts, said peptide representing the peptide unit which is repeated 8 times in 8ΔET [16-29]. The higher capacity obtained through immobilization of 8ΔET [16-29] stems from the fact that just some of the eight peptide chains take part in the covalent interaction with the solid phase, whereas the other ones are free to interact non-covalently with the Big ET. On the contrary, in the case of the column prepared with the employment of ΔET [16-29], the peptide is linked to the solid phase either through the N-terminal alpha-amino group or through the epsilon-amino groups of the lysines present in the positions 2 and 13 in the peptide sequence. In both cases, the peptide is not homogeneously immobilized, and anyway the closeness to the solid phase can cause remarkable decreases in the binding capacity for the Big ET. Such effects are quite common indeed also for other affinity systems and anyway they are largely illustrated in the literature. The usefulness of the present invention can be further appreciated, as is disclosed in the following examples, in the case of realizing analytical methodologies for the quantitative determination of said Big ET.

According to a particular embodiment of the present invention, the peptide 8ΔET [16-29] can be immobilized non-covalently on suitable plastic microcontainers which are then treated with proper protein solutions in order to remove the presence of any possible non-specific interaction sites, and then they are washed employing suitable solutions. Big Endothelin [1-38] or [16-29], labelled with suitable reactants, can then be added to each microcontainer, the whole being then kept under incubation in order to cause the interaction to occur. The container can then be washed in order to remove the excess Big Endothelin [1-38] or [16-29] that has not reacted, and then they are examined to determine the amount of Big ET or of its derivative [16-29] that has reacted. The technical literature largely disclosed both the labelling methods, which can be realized through the introduction of radioisotopes, enzymes, fluorescent or chromophore compounds, or biotin, and the methods for the determination of such labelled compounds, and all said methods are well known to those who are skilled in the art. The strength of the non-specific interaction can be determined by incubating Big Endothelin [1-38], labelled in the presence of unlabelled excess Big ET, and analyzing the data obtained according to Scatchard's procedure. The specificity of interaction between the peptides which are the object of this invention can be determined through incubation within microcontainers derivatized with 8ΔET [16-29] with different peptides and proteins labelled in the presence or in the absence of unlabelled material. Further indications about

specificity can be obtained through incubation of the fragments 16-21 or 22-32 of Big Endothelin [1-38] which have a very low affinity towards the peptide 8ΔET [16-29]. Similarly to the case of the peptide 8ΔET [16-29], the peptide 8ΔTNF [144-157] has been immobilized both on a solid support for the preparation of affinity columns for the purification of TNFα , both on microplates for realizing a test to determine TNF quantitatively.

The usefulness of this invention is also evident, as illustrated in the examples 5, 7 and 8, in the case when the micromolecule of interest to be purified or to be analyzed contains more than just one site with the same sequence idropathically complementary to the immobilized multimeric ligand. In that case, the polyvalence of the multimeric ligand is characterized by an increase in the binding affinity for the molecule of interest. A general situation in the application of the present procedure can occur in the case of proteins made up of different sub-units which are all equal as regards the amino acid sequence and the structure. A peptide which is hydropathically complementary to an exposed site of such proteins can be synthesized according to the process which is the object of this invention in the octameric form, immobilized on a solid phase, and employed for purifying or for determining quantitatively the oligomeric protein of interest. The presence of a number of binding sites increases indeed the affinity exponentially, as in the case of bivalent antibodies which is largely disclosed in the literature. The observed increase of the binding affinity of the complementary multimeric peptides is applied clearly not only to the field of purifications carried out through the affinity chromatographic method or to the field of realization of analytical procedures. Indeed, as is evident to all those who are skilled in the art, the possibility of creating synthetic molecules which are able to become associated to protein macromolecules (proteins, receptors, antibodies, and so on) with high affinity (i.e. having low values of the dissociation constants) is largely exploited in the pharmacological field, for designing new synthetic drugs having activities closely directed towards a particular macro-molecule having important biological function. A particular advantage in the use of such multimeric compounds with respect to the use of single peptides, is to be found both in the increased affinity, and in the higher stability with respect to the action of the proteolytic enzymes which are normally present in biological fluids. Indeed, the high molecular weight that results from the union of many peptide units gives the compound a higher steric inaccessibility as regards the active proteolysis site of the enzymes. Moreover, it is evident to those who are skilled in the art that it is possible to change, according to the various cases and the various specific applications, both the number of peptide units linked to the polylysine nucleus, and to introduce further residues in order to further space the peptide chains apart, without departing from the objects of the present invention.

EXAMPLE 1

Solid-phase synthesis of 8ΔET [16-29]

The sequence of the peptide hydropathically complementary to the sequence 16-29 of the human Big Endothelin [1-38] (HLDIIWVNTPEHIV) has been obtained on the basis of the method AMINOMAT previously disclosed by the same authors (Italian Patent Application No. 20755 A/90) and called ΔET [16-29] (RKFLAGLRARRLKF). The peptide ΔET [16-29] has been then synthesized by means of a solid-phase synthesis, employing a Fmoc methodology and exploiting standard coupling procedures of the single amino acid through the formation of active esters with dicyclohexylcarbodiimide/hydroxybenzotriazole [DCC/HOBt]. Similarly, the sequence 16-29 of the Big Endothelin has been synthesized. The peptides after the synthesis have been subjected to the removal of the protection groups and have been detached from the solid support according to standard procedures, and purified until obtaining chromatographic homogeneity by means of RP-HPLC. The octadentate polylysine nucleus employed for synthesizing the multiple peptide 8ΔET [16-29] (Figure 1A) has been obtained through successive coupling operation in three cycles of alpha-Fmoc-Lysine (epsilon-Fmoc) on the initial solid support (resin Fmoc-Glycine-HMP). The sequence ΔET [16-29] has been then synthesized on the initial polylysine nucleus (Figure 1B) as disclosed previously. The multiple peptide, after removal the protection groups and after detaching the same from the resin, has been purified from the low molecular weight contaminants by dialysis against 0.1 M acetic acid for two days. Finally the dialyzed material has been frozen and lyophilized. Following the same scheme, the peptides ET [16-29] and 8ET [16-29] have also been prepared, whose structure is given respectively in Figures 1C and D.

EXAMPLE 2

Preparation od solid phase for affinity chromatography

The peptides ΔET [16-29] and 8ΔET [16-29] have immobilized on a support previously activated through the introduction of epoxide groups (EUPERGIT C30 N), employing the same peptide/support ratio. The peptides 8ΔET [16-29] (2 mg) and ΔET [16-29] (2 mg) have been dissolved separately in 10 ml of 0.1 M $NaH_2CO_3$, 0.5 M NaCl, pH 8.5, and added separately to two aliquots of 1 g of EUPERGIT C30 N. The mixtures have been incubated for 24 hours under stirring at room temperature. The introduction of the peptides within the resin has been followed by means of

RP-HPLC, and after 24 hours an amount of about 80 % of the peptide initially present in the two preparations turned out to be covalently linked to the support. Such incorporation percentage has been then confirmed by analysis of amino acids, which analysis were carried out on weighed amounts of the two derivatized resins.

The two derivatized peptide supports were employed for filling two glass columns for affinity chromatography (80 x 6.6 mm I.D., total volume 2.3 ml ) that were equilibrated with 0.1 M TRIS∗HCL, at pH 6.8 and with a flowrate of 1 ml/min. The eluent was continuously monitored by means of optical absorption determinations at 280 nm wavelength. For determining the capacity of the columns, 500 microlitres of ET [16-29] dissolved at a concentration of 1 mg/ml in the elution buffer were injected. Under the same elution conditions, the column prepared with the peptide 8ΔET [16-29] (Figure 2A) retains the injected material fully, whereas the column prepared with ΔET [16-29] retains just 50 % of the same (Figure 2B).

EXAMPLE 3

Preparation and purification of Biotin ET [1-32]

The labelling of ET [1-32] for realizing analytical tests on plastic microcontainers was carried out through derivatization of the peptide with biotin according to the following procedure:

1) 2.5 mg of ET [1-32] was dissolved in 1 ml of 20 mM NaAc, at pH 6.0, and 400 microl of a solution at a concentration of 2 mg/ml of biotin aminocaproate-N-hydroxysuccinimide ester in ethanol/water 1/1 was added. The mixture was then incubated under stirring for 5 hours at room temperature.
2) The reaction mixture was then diluted 1/1 with 50 mM TRIS, at pH 6.8, and 1 ml was then injected into the affinity column prepared with the peptide 8ΔET [16-29] and put in equilibrium with 50 mM TRIS at pH 6.8, at a flowrate of 1 ml/min. The effluent was then monitored by recording the optical absorption at 280 nm. After complete elution of the material that had not been kept and was made up of the excess reagent as well as of reaction by-products, the eluent was changed to 0.1 M HAc, pH 3.0, as shown in Figure 3B. The purified product, called Biotin ET [1-32] was then freeze-dried and employed directly for the successive objects.

EXAMPLE 4

Interaction of biotin-ET [1-32] with 8ΔET [16-29]-sensitized microplates

Some 96-well polystyrene plates (NUNC) have been sensitized with 100 microl/well of 8ΔET [16-29] solutions in a 0.1 M sodium carbonate buffer at pH 9.6, at various concentrations (2.0, 0.5, 0.2, 0.1, 0.0 microg/ml) for one night at 4°C. The plates were then washed three times with a 0.15 M sodium chloride, 0.5 M sodium phosphate solution at pH 7.3 (PBS) through repeated filling and emptying operations. At the end of such operations, each well was filled with 200 microl of a bovine serum albumin (BSA) solution containing 3 % PBS, and then incubated for 2 hours at room temperature. The plates were then washed again for three times with PBS, and each well was filled with 100 microl of a Biotin-ET [1-32] solution at various concentrations (0.2, 0.1, 0.05, 0.0 microg/ml) in PBS diluted at the ratio of 1/1 with distilled water containing 0.5 % BSA (PES-B) this operation being followed by incubation for 2 hours at room temperature. At the end of the operations the plates were washed again with PBS (8 times) and filled with 100 microl/well of a streptavidin-peroxidase solution (Sigma Chemical Company), diluted in the ratio of 1/1,000 with PBS-B containing 0.05 % Tween 20. After one-hour incubation at 37°C, the plates were washed with PBS 8 times and filled with 100 microl/well of a 1 mg/ml o-phenilendiamine solution in 0.1 M sodium citrate buffer at pH 5 containing 5 mM hydrogen peroxide. The chromogenic reaction was carried out for 30 minutes at 37°C and stopped through the addition of 25 microl. of 4.5 M sulfuric acid. The optical density of each well was then determined by means of a microplate reader (Model 3550 Microplate Reader, Biorad). The results obtained which are shown in Figure 3, pointed out the effective binding between the Biotin-ET [1-32] and the 8ΔET [16-289]-sensitized wells. The binding, as expected, turned out to be dependent on both the Biotin-ET [1-32] and the 8ΔET [16-29] concentrations. More particularly, the employment of 8ΔET [16-29] at the concentration of 0.5 mg/ml in the step of sensitization of the plates and Biotin-ET [1-32] at the concentration of 0.2 microg/ml seems to be sufficient to generate a measurable signal for developing a competitive test with ET [1-32].

EXAMPLE 5

Test of competitive binding for ET [1-32]

In order to evaluate the specificity of the bond between Biotin-ET [1-32] and the 8ΔET [16-29]-sensitized wells, as

disclosed in example 4, a binding competition test of Biotin-ET [1-32] with ET [1-32] and with two negative-control peptides C1 and C2 was carried out, the binding occuring with 8ΔET [16-29]-coated wells. The experiment was carried out following the procedure disclosed n example 4 with fixed concentrations of 8ΔET [16-29] (0.5 microg/ml) and of Biotin-ET [1-32] (0.2 microg/ml) and in the presence of various concentrations of competitor peptides between 1 and 1,000 microg/ml.

The results obtained which are shown in Figure 5, point out the effective competition of the binding between Biotin-ET [1-32] and 8ΔET [16-29] with ET [1-32] at concentrations between 5 and 250 microg/ml but not with C1 and C2. This suggests that the observed interaction between the complementary peptides is specific. The specificity of the interaction was further confirmed through competition experiments with ET [16-21]. In that case no appreciable binding competition was observed at the concentrations employed in the tests. This can be easily interpretated in terms of drastic reduction in the affinity due to the lack of 11 residues in the hydropathically complementary region which are necessary for stabilizing the interaction. It was observed, in agreement with such hypothesis, that the peptide ET [16-29], containing the whole complementary region is on the contrary capable of competition. As a conclusion, the binding between Biotin-ET [1-32]and 8ΔET [16-29] observed on the polystyrene plates is specific and it depends on the hydropathically complementary regions of the two peptides, and is exploitable for the determination of molecules containing said regions, as for instance Big-Endothelin and pro-Endothelin.

EXAMPLE 6

Determination of the relative affinity of ET [16-29] and 8ET [16-32] towards 8ΔET [16-29]

The column containing the peptide 8ΔET [16-29], prepared as disclosed in example 2, was put in equilibrium with 1M $NH_4Ac$ at pH 5,7, at a flowrate of 1.0ml/ min. Uncer such conditions of high ionic strength, the interactions between complementary peptides turn out to be remarkable reduced as disclosed in the literature (Fassina et al., J. Biol. Chem. 264: 11252, 1989) and it is possible to observe just a delay in the elution volume of the interacting compound with respect to the dead volume of the column instead of a full adsorption. It is possible the calculate from the elution volume the interaction dissociation constant Km/p on the basis of the equation:

$$\frac{1}{V-V_o} = \frac{K_{M/P}}{M_T} + \frac{[P]}{M_T}$$

wherein V is the elution volume, $V_o$ is the dead volume of the column, $M_t$ is the column capacity and P is the amount of the eluted component (Fassina and Chaiken, Adv. in Chromatogr., 27:247, 1987). In such conditions, the peptide ET [16-29] undergoes an elution delay equal to 3 ml (Figure 6A) whereas the peptide SET [16-29] is not eluted even after 100 minutes, and it is necessary to change the eluent to 0.1 M HAc for the complete elution of the same (Figure 6B). The dissociation constant of the peptide ET [16-29] thus turns out to be at least 50 times as high with respect to that obtained with the peptide 8ΔET [16-29]. ]. This means that the polyvalence in the interaction between 8ΔET [16-29] and 8ET [16-29] increases the association extent by about 2 orders of magnitude.

EXAMPLE 7

Design and solid-phase synthesis of 8ΔTNF [144-157]

The sequence of the peptide hydropathically complementary to the sequence 144-157 of the Tumor Nerosis Factor (TNFalpha) ( FAESGQVYFGIIAL) has been obtained on the basis of the AMINOMAT procedure which has been disclosed previously (Italian Patent Applications No. 21396 A/89 and No. 20755 A/90) and called ΔTNF [144-157] [DYLAGFKAHGKKYR]. The peptide 8ΔTNF [144-157] was then synthesized by solid-phase synthesis with Fmoc methodology starting from an octadentate polylysine nucleus provided with glycine and arginine spacers as illustrated in Figure 7A. Similarly to what has been disclosed previously, the peptide 8ΔTNF [144-157] (Figure 7B) was then deprotected, detached from the resin and purified through dialysis against 0.1 M acetic acid for two days. Finally the dialyzate was frozen and freeze-dried. The peptide 8ΔTNF [144-157] (5 mg) was then immobilized on a previously activated support (1 g) with epoxide groups (EUPERGIT C30 N), exploiting the same conditions as those reported previously. The derivatized peptide support was then employed for filling a glass column for affinity chromatography (80 x 6.6 mm I.D., total volume 2.3 ml), which was then equilibrated with 0.1 M TRIS HCl at pH 6.8 at a flowrate of 2.0 ml/min. In order to determin the recognition properties of the column towards TNF alpha, injections were made of 100 microl of TNF alpha at a concentration of 0.5 mg/ml dissolved in the elution buffer. After about 10 minutes the buffer was changed to 0.1 M acetic acid in order to allow the elution of the adsorbed material to occur (Figure 8A). The material eluted with

acetic acid was then analyzed by RP-HPLC to confirm the occurrence of the recognition between TNF alpha and 8ΔTNF [144-157] (Fig. 8B).

EXAMPLE 8

Interaction between Biotin TNF and 8ΔTNF [144-157]-sensitized microplates

50 microl of a solution of 8ΔTNF[144-157] at a concentration of 0.04 mg/ml were added to each well of a 96-well, PVC plates, in a 0.1 M sodium bicarbonate buffer at pH 9.6. The plates were then incubated for 1 hour at room temperature and then washed three times with PBS. Each well was then filled with 200 microl of 3 % bovine serum albumin (BSA) solution in 0.025 M sodium phosphate buffer at pH 6.5, containing 0.075 M NaCl (PBS). After one-hour incubation at room temperature, the plates were washed with PBS and filled with TNF/TNF-Biotin mixtures at various delutions in 1 % w/v PBS-BSA, containing 0.05 % v/v Tween 20, 10 KIU/ml Aprotinine, PMSF 10 microM, EDTA 10 microM, (PBS-TAPE) in the presence and in the absence of 1 % normal goat serum. The plates were then incubated again for 1 hour at room temperature and then washed with PES. Each well was then filled with 100 microl of a streptavidin-peroxidase solution (Sigma) ciluted 1/2,000 with PBS-TAPE and incubated for 1 hour at room temperature. Finally the plates were washed, filled with a chromogen ABTS (KPL) solution (100 microl/well) and incubated for 30 minutes at room temperature. The absorbance at 405 nm of each well was then determined by means of a microplate reader (BioRad Model 25550 EIA Reader). As shown in minute 8,the binding between 8ΔTNF [144-157] immobilized on the wells and TNF alpha-Biotin turns out to be specific and it can be in competition with TNF alpha both in the presence and in the absence of goat normal serum.

EXAMPLE 9

Determination of the proteolysis kinetics of the substrate ET (16-32) and pro-endothelin by means of alpha-chymotrypsin

The peptide pro-endothelin 1-38 was obtained from commercial sources (Novabiochem, CH). Solutions of such peptide and of the peptide ET 16-32 at a concentration of 0.1 mg/ml were prepared in 50 mM TRIS at pH 6.8, to which the addition was carried out of alpha-chymotrypsin aliquots, so obtaining an enzyme/substrate ratio of 1/1,000. The proteolysis reaction was followed in time by sampling after different incubation times at 20°C, aliquots of 50 microl for chromatographic analysis by means of RP-HPLC, employing a reverse -phase column ABI RP-300 30 x 2.1 mm I.D., equilibrated with $H_2O/CH_3CN/TFA$ 97/3/C.1 at a flowrate of 0.5 ml/min, and employing a linear elution gradient of the type:

| Time | $CH_3CN$ |
|---|---|
| 0 | 3 |
| 5 | 3 |
| 25 | 35 |
| 30 | 80 |
| 35 | 80 |
| 37 | 3 |

and monitoring the eluent by 225 nm wavelength. Under such conditions, the elution times of the peptides and of the relative fragments were:

| Peptide | $T_{elution}$ (min) |
|---|---|
| ET (16-32) | 23 |
| ET (1-38) | 32 |
| ET (1-21) | 22 |
| ET (22-38) | 15 |
| ET (16-21) | 21.5 |
| ET (22-32) | 12 |

The degradation percentages were then calculated according to the formula:

$$\% \text{ degradation} = \frac{A_{T=0} - A_{T=x}}{A_{T=0}} \times 100$$

wherein $A_{T=0}$ corresponds to the area under the RP-HPLC peak relative to ET (16-32) or ET (1-38) in the absence of chymotrypsin, whereas $A_{T=x}$ corresponds to the area regarding the fragments ET (16-21) or ET (1-21) determined after a time x of treatment with alpha-chymotrypsin at 20°C. The degradation kinetics data of the two peptides ET (16-32) and pro-ET (1-38) are shown in Figure 10. The close correspondence of the two kinetic curves obtained for the degradation of the two peptides allows to set forth that the fragment 16-32 of pro-endothelin is an optimal alternative substrate for stucying the action of pro-endothelin specific proteolytic enzymes.

EXAMPLE 10

Test for detecting pro-endothelin specific proteolytic activities present in biological fluids

A) Preparation and purification of Biotin ET (16-32). The labelling of ET (16-32) for realizing analytical tests on plastic microcontainers was carried out derivatizing the peptide with biotin according to the following procedure:

1) 2.5 mg of ET (16-32) was dissolved in 1 ml of 20 mM NaAc, at pH 6.0, and 400 microl of a solution of biotin aminocaproate-N-hydroxysuccinimide ester at a concentration of 2 mg/ml in ethanol/water 1/1 was added. The mixture was then incubated under stirring for 5 hours at room temperature.
2) The reaction mixture was then diluted 1/1 with 50 mM TRIS at pH 6.8, and 1 ml was then injected into an affinity column prepared with the peptide 8ΔET (16-29). The purified product, which is called Biotin-ET (16-32) was then freeze-dried and employed directly for the next steps.

B) Preparation of sensitized plates

Some 96-well polystyrene plates (NUNC) were sensitized with 100 microl/well of a solutioon at a concentration of 200 microg/ml of 8ΔET (16-29) in 0.1 M sodium carbonate buffer at 9.6 for one hour at 20°C. The plates were then washed three times with a 0.15 M sodium chloride solution, 0.5 % sodium phosphate at pH 6.5 (PBS), through repeated filling and emptying operations. At the end of the operations, each well was filled with 200 microl of a 3 % bovine serum albumin (BSA) solution in PBS containing 0.1 mM PMSF, 0.1 mM EDTA, aprotinin 50 microg/ml and incubated for one hour at room temperature. The plates were then washed again 8 times with PBS.

C) Standard treatment

Each well was filled with 100 microl of a Biotin-ET (16-32) solution at various concentrations (10; 1; 0.1; 0.001 and 0.0 microg/ml) in PBS diluted in the ratio of 1/1 with distilled water containing 0.5 % BSA (PBS-B) and previously treated with variable concentration of alpha-chymotrypsin (10; 5; 1; 0.1; 0.0 microg/ml) and incubated for 1 hour at room temperature.

D)Development of plates

At the end of the operations, the plates were filled with 100 microl per well of a streptavidin-peroxidase solution (Sigma Chemical Company) diluted in the ratio of 0/1,000 with PES-B containing Tween 2C at 0.05 % concentration. After one hour of incubation at room temperature, the plates were washed with PBS (8 times) and filled with 100 microl/well of 1 mg/ml o-phenylendiamine solution in 0.1 M sodium citrate buffer at pH 5, containing 6 mM hydrogen peroxide. The chromogenic reaction was carried out for 30 minutes at 20°C and then stopped by the addition of 100 microl of 0.1 M hydrochloric acid. The optical density of each well was then measured by means of a microplate reader (BioRad Model 3550 Microplate Reader). The results so obtained shown in Figure 11 pointed out that Biotin-ET (16-32) binds to the sensitized wells in a way proportional to its concentration, whereas if it is previously treated with chymotrypsin, it looses such b nding capability. Hence it is immediately clear that the presence of enzymes capable of proteolysing ET (16-32) is easily detectable by incubating the sample under test with a standard Biotin-ET (16-32) solution and measuring as disclosed previously the amount of Biotin-ET (16-32) linked to the sensitized wells.

EXAMPLE 11

Test for detecting inhibitors of pro-endothelin specific proteolytic activities in biological fluids

A) Preparation of sensitized plates

Some 96-well plates were sensitized with the 8ΔET (16-29) peptide as disclosed previously in Example 10.

B) Standard treatment

Samples containing inhibitors of pro-endothelin specific proteolytic activities (100 microl) were added to 50 microl of a solution at a concentration of 1 microg/ml of alpha-chymotrypsin dissolved in 0.5 % BSA PBS at pH 6.8 and next 50 microl of a solution of biotin-ET (16-32) at the concentration of 1 microg/ml was added. 100 microl of such mixture was then added to the sensitized wells after 1 hour incubation period.

C) Development of plates

The development was carried out as disclosed previously in Example 10.

Data obtained and shown in Figure 12 point out clearly that the presence of inhibitors in the analyzed sample of pro-endothelin specific enzymes is easily evidenced by the positive reading values of the wells. When the degradation of biotin-ET (16-32) is inhibited, the amount of the biotinized substrate capable of binding to the wells is higher than that of samples that do not have such inhibiting activity.

**Claims**

1. Nonlinear peptides hydropathically complementary to peptides or protein portion with known amino acid sequence, said peptides being characterized in that they comprise:

   an amino acid nucleus comprising at least an amino acid with at least two terminal amino functions,
   a linear amino acid sequence which is complementary to said known amino acid sequence and is bonded through a carbamido link to each one of said terminal amino functions.

2. Nonlinear peptides according to claim 1, wherein said hydropathically complementary amino acid sequence consists of a number of amino acids between 10 and 20.

3. Nonlinear peptides according to anyone of the preceding claims wherein said amino acid nucleus in the central position has the following formula

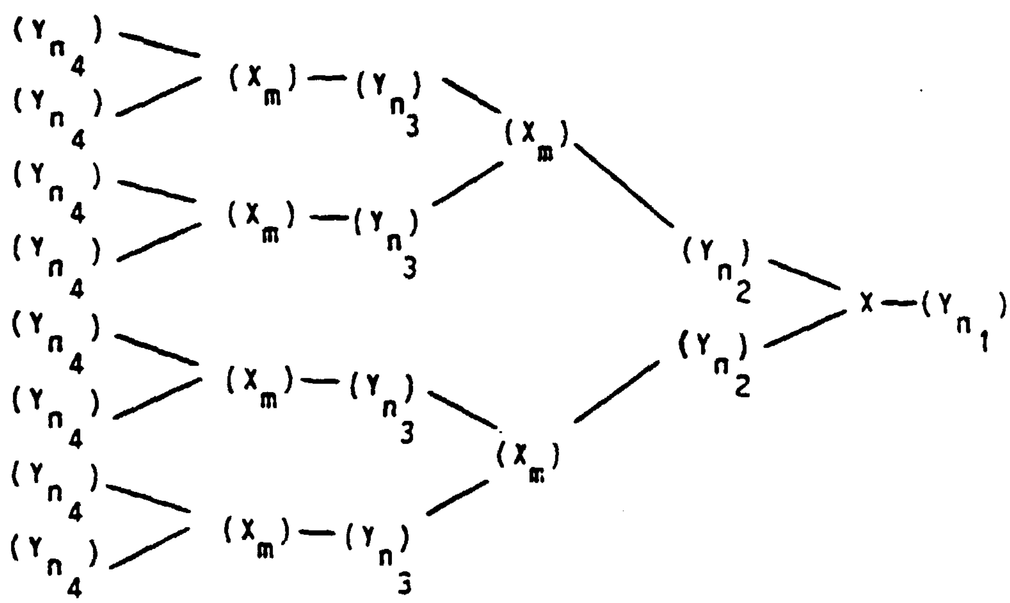

   wherein X is an amino acid having at least two amino functional residues; Y is an amino acid selected from the group of alanine, glycine or arginine; m is a number in the range from 0 and 1; $n_1$, $n_2$, $n_3$ and $n_4$ are numbers between 0 and 10 and wherein the bonds are carbamido bonds.

4. Nonlinear peptides according to anyone of the preceding claims wherein said amino acid with at least two terminal amino functions (X) is lysine.

5. Nonlinear peptides according to anyone of the preceding claims 1-3 wherein said amino acid having at least two terminal amino functions (Y) is ornitine.

6. Nonlinear peptides according to anyone of the preceding claims, wherein said amino acid sequence is hydropathically complementary to a portion of the Big Endothelin.

7. Nonlinear peptides according to claim 6 wherein said sequence is hydropathically complementary to the portion of the Big Endothelin from the amino acid 16 to the amino acid 29.

8. Nonlinear peptides according claim 7 wherein said sequence has the following formula

RKFLAGLRARRLKF.

9. Nonlinear peptides according to anyone of the preceding claims 1-5 wherein said amino acid sequence is hydropathically complementary to a TNF alpha portion.

10. Nonlinear peptides according to claim 9 wherein said sequence is hydropathically complementary to the portion 144-157 of TNF alpha.

11. Nonlinear peptides according to claim 10 wherein said sequence has the following formula

DYLAGFKAHGKKYR.

12. A process for synthesizing nonlinear peptides according to anyone of the preceding claims, said process comprising the steps of:

- immobilizing on a solid phase the carboxyl residue of an amino acid
- causing a carbamido bond to form between the amino residue of said amino acid and the carboxyl residue of an amino acid having at least two terminal amino functions
- adding to each one of said terminal amino functions and amino acid which has itself at least two terminal amino functions,
- repeating such step from 0 to 2 times
- synthesizing linear peptides covalently linked to each one of said terminal amino functions.

13. A process according to claim 12, wherein amino acids selected from the group of glycine, alanine or arginine are interposed so as to act as spacers between the amino acids having at least two terminal amino functions.

14. A process according to claim 12, wherein said peptides are hydropathically complementary to at least one portion of said Big Endothelin.

15. A process according to claim 12, wherein said peptides are hydropathically complementary to at least one portion of said TNF alpha.

16. A process for the preparation of affinity chromatography columns employing nonlinear peptides according to anyone of the preceding claims 1-11, comprising the steps of:

- immobilizing the derivatized support on a chromatographic column and putting such column in equilibrium with a saline buffer
- injecting into the column an amount of a raw compound containing the protein or the peptide to be purified, said amount being compatible with the separation power of the column
- washing the column with an elution buffer till the foreign matter disappear, and eluting said protein or said peptide linked to the column by means of an eluent change so as to oppose the binding.

17. A process according to claim 16, wherein said protein to be purified is Big Endothelin.

18. A process according to claim 16, wherein said protein to be purified is the Tumor Necrosis Factor (TNF alpha).

19. A process for the determination of peptides or proteins both of natural and of recombinant origin in samples, said process comprising:

- the treatment of a container divided into compartments with nonlinear peptides according to anyone of the preceding claims 1-11
- labelling a purified aliquot of the peptide or of the protein to be determined
- incubating said labelled peptide or protein within said container
- incubating the sample containing said peptide or protein to be determined in said container
- detecting the presence of said peptide or protein to be determined in said sample by means of differential determinations.

**20.** A process according to claim 19, wherein said labelling is carried out with radioisotopes or through enzymic reactions or with a fluorescent or chromophoric group or with biotin.

**21.** A process according to claims 18 or 19, wherein said protein to be determined is Big Endothelin.

**22.** A process according to claims 18 or 19 wherein said protein is the Tumor Necrosis Factor (TNF alpha).

**23.** A process for the identification of proteolytic activities and/or of inhibitors of such activities, said process comprising:

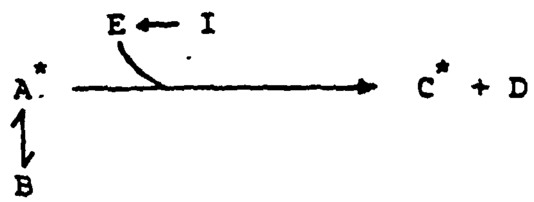


wherein

E represents said proteolytic activity which is capable of catalyzing the conversion of a substrate A into the fragments C and D, said substrate A comprising at least a protein fragment labelled with at least a proteolytic attack site,
I represents said inhibiting activity towards E,
B represents a ligand which is capable of recognizing said substrate A but not said fragments C and D; B consisting of molecules made up of amino acid nucleus comprising at least an amino acid having at least two terminal amino functions and a linear amino acid sequence which is hydropathically complementary to said protein fragment, and is bound through a carbamido bond to each one of said terminal amino functions;
* points out that said substrate A is labelled.

**24.** A process for the identification of proteolytic activities and/or of inhibitors of such activities according to claim 23, wherein said proteolytic activities and/or activities that inhibit the same are present in biological fluids or in fermentation broths or cell extracts.

**25.** A process for the identification of proteolytic activities and/or of activities capable of inhibiting the same according to anyone of the preceding claims 23 or 24 wherein said protein fragment is labelled by means of radioisotopes, or of enzymes, or of fluorescent compounds or of chromophores or of biotin.

**26.** A process for the identification of proteolytic activities and/or inhibitors of such activities according to claim 25, wherein said protein fragment is labelled by means of biotin derivatization and is evidenced by incubation with streptavidin conjugated with peroxidase.

**27.** A process for the identification of proteolytic activities and/or inhibitors of such activities according to anyone of the preceding claims 23-26, said process comprising also means for the separation of the complex protein-ligand fragment from the protein fragments which are not bound and from the proteolyzed derivatives.

**28.** A process for the identification of proteolytic activities and/or inhibitors of such activities according to claim 27 wherein said separation means are realized by immobilizing in the solid phase said ligand and washing said unbound. protein fragments and said proteolyzed derivatives away.

**29.** A process for the identification of proteolytic activities and/or inhibitors of such activities according to claim 28 wherein said solid phase comprises plastic plates divided into compartments.

**30.** A process for the identification of proteolytic activities and/or inhibitors of such activities according to anyone of the preceding claims 27-29 comprising the step of determining said labelled protein fragments which are bonded to said ligand immobilized on a solid phase.

**31.** A process for the identification of proteolytic activities and/or inhibitors of such activities according to claims 23-30 wherein said substrate comprises a fragment of pro-endothelin and said ligand comprises amino acid sequences which are hydropathically complementary to said fragment of pro-endothelin.

**32.** A process for the identification of proteolytic activities and/or inhibitors of such activities according to claim 31 wherein said fragment of pro-endothelin is made up of the sequence from the amino acid 16 to the amino acid 32, having the formula

HLDIIWVNTPEHIVPYG

and said ligand is the molecule 8ΔET (16-29).

**33.** A process for the identification of proteolytic activities and/or inhibitors of such activities according tpo claim 32 wherein in said proteolytic attack site is between the amino acids 21 and 22 of said fragment 16-32 of pro-endothelin, such as to generate in the presence of proteolytic activities two fragments ET (16-21) and ET (22-32) which are uncapable of binding to said molecule 8ΔET (16-29).

**Patentansprüche**

**1.** Nicht lineare Peptide, die hydropatisch komplementär zu Peptiden oder Proteinteilen mit bekannten Aminosäurefrequenzen sind, dadurch gekennzeichnet, dass sie:

einen Aminosäurekem, welcher mindestens eine Aminosäure mit mindestens zwei Endaminofunktionen enthält

eine lineare Aminosäurezequenz, die zur vorgenannten bekannten Aminosäurezequenz komplementär und durch eine Karbonil-Kuppelung an je eine der vorgenannte Aminofunktionen gebunden ist, enthalten.

**2.** Nicht lineare Peptide nach Anspruch 1, worin die vorgenannte hydropatisch komplementäre Aminosäurezequenz aus einer Anzahl von Aminosäuren zwischen 10 und 20 besteht.

**3.** Nicht lineare Peptide nach je einem der vorhergehenden Ansprüche, worin der vorgenannte Aminosäurekem im Mittelteil die folgende Formel hat:

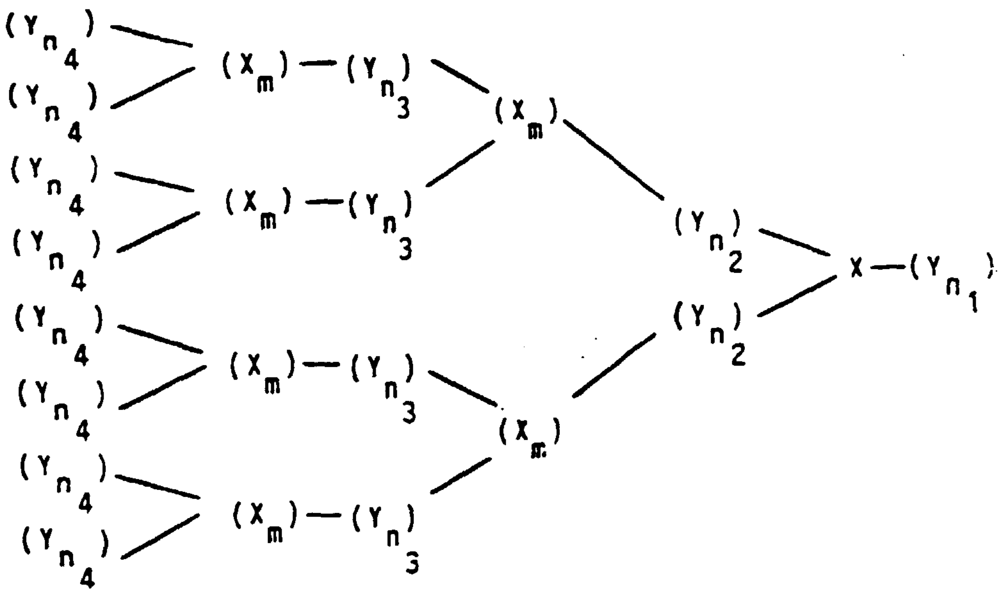

wobei

X eine Aminosäure mit mindestens zwei Aminofunktioresten ist; Y eine von einer aus Alanin, Glyzin or Arginin bestehenden Gruppe ausgewählte Aminosäure ist;
m eine Zahl zwischen 0 und 1 ist; $n_1$, $n_2$, $n_3$ und $n_4$ eine Zahl zwischen 0 und 10 sind und wobei die Kuppelunge Karbamidkuppelunge sind.

**4.** Nicht lineare Peptide nach je einem der vorhergehenden Ansprüche, worin die vorgenannte Aminosäure mit mindestens zwei Endaminofunktionen (X) Lysin ist.

**5.** Nicht lineare Peptide nach je einem der vorhergehenden Ansprüche 1 bis 3, worin die vorgenannte Aminosäure mit mindestens zwei Endaminofunktionen (Y) Omithin ist.

**6.** Nicht lineare Peptide nach je einem der vorhergehenden Ansprüche, worin die vorgenannte Aminosäuresequenz komplementär zu einem Anteil von "Big-Endothelin" ist.

**7.** Nicht lineare Peptide nach Anspruch 6, worin die vorgenannte Sequenz hydropatisch komplementär zu einem Anteil von "Big-Endothelin" der Aminosäure 16 bis 29 ist.

**8.** Nicht lineare Peptide nach Anspruch 7, worin die vorgenannte Sequenz die folgende Formel hat:

RKFLAGLRARRLKF.

**9.** Nicht lineare Peptide nach Ansprüche 1-5, worin die vorgenannte Aminosäuresequenz hydropatisch komplementär zu einem TNF-Alphaanteil ist.

**10.** Nicht lineare Peptide nach Anspruche 9, worin die vorgenannte sequenz hydropatisch komplementär zum Anteil 144-157 von TNT-Alpha ist.

**11.** Nicht lineare Peptide nach Anspruche 10, worin die vorgenannte Sequenz die folgende Formel hat:

DYLAGFKAHGKKYR.

**12.** Verfahren zur Synthese von nicht linearen Peptiden nach je einem der vorgehenden Ansprüche, mit folgenden Verfahrensschritten:

- Immobilisierung des Karboxylrestes einer Aminosäure auf einen festen Phase;
- Bildung einer Karbamidbindung, zwischen dem Aminorest der vorgnannten Aminosäure und dem Karboxylrest einer Aminosäure mit mindestens zwei Endaminofunktionen;
- Zugabe zu einer der vorgenannten Endaminofunktionen einer Aminosäure, die selbst mindestens zwei Endaminofunktione hat;
- Wiederholung dieses Verfahrensschrittes von 0 bis 2 Mal;
- Synthese von linearen Peptiden, die kovalent mit je einer der vorgenannten Endaminofunktionen gebunden sind.

**13.** Verfahren nach Anspruch 12, worin die aus der Gruppe von Glyzin, Alanin oder Arginin ausgewählten Aminosäuren so angeordnet sind, dass sie als Abstandhalter zwischen den Aminosäuren mit mindestens zwei Endaminofunktionen wirken.

**14.** Verfahren nach Anspruch 12, worin die vorgenannten Peptiden hydropatisch komplementär zu mindestens einem Anteil des vorgenannten Big-Endothelins sind.

**15.** Verfahren nach Anspruch 12, worin die vorgenannten Peptiden hydropatisch komplementär zu mindestens einem Anteil vom vorgenannten TNF Alpha sind.

**16.** Verfahren zur Vorbereitung von Affinitäts-Chromatographie-Säulen durch Anwendung von nicht linearen Peptiden nach je einem der vorhergehenden Ansprüche 1 bis 11, worin folgende Verfahrensschritte vorgesehen sind:

- Immobilisierung der derivatizierten Auflage auf einer chromatographischen Säule und die vorgenannte Säule ins Gleichgewicht mit einem Salzpuffer bringen;
- Einspritzung in die Säule eine Menge einer Rohverbindung, die das zu reinigende Protein oder Peptin enthält, wobei diese Menge mit der Abscheidekraft der Säule verträglich ist;
- Abwaschen der Säule mit einem Elutionspuffer bis zur Beseitigung von Aussenstoffen und Elution des vorgenannten an die Säule gekoppeten Proteins oder Peptids durch einen Austausch des Elementen, um so der Bindung entgegen zu wirken.

**17.** Verfahren nach Anspruch 16, worin das zu reinigende Protein Big-Endothelin ist.

**18.** Verfahren nach Anspruch 16, worin das vorgenannte zu reinigende Protein der Tumor Necrosis Factor (TNF-Alpha) ist.

**19.** Verfahren zur Bestimmung von Peptiden oder Proteinen natürlicher und rekombinanter Herkunft in Proben, worin folgende Verfahrensschritte vorgesen sind:

- Behandlung eines Behälters, der in Abteilungen mit non linearen Peptiden nach je einem der vorhergehenden Ansprüche 1 bis 11 getrennt ist;
- Etikettierung eines gereinigten Anteils des zu bestimmenden Peptids oder Proteins;
- Inkubation des etikettierten Peptids oder Proteins im vorgenannten Behälter;
- Inkubation der das vorgenante zu bestimmende Peptid order Protein enthaltenden Probe im vorgenannten Behälter;
- Ermittlung der Anwesenheit des vorgenannten zu bestimmenden Peptids oder Proteins in der vorgenannten Probe durch differentiale Bestimmungen.

**20.** Verfahren nach Anspruch 19, worin die vorgenannte Etikettierung mit Radioisotopen oder durch enzymetrische Reaktionen oder mit einer fluoreszenten oder chromophorischen Gruppe, oder mit Biotin durchgeführt wird.

**21.** Verfahren nach Anspruch 18 oder 19, worin das zu bestimmende Protein Big Endothelin ist.

**22.** Verfahren nach Anspruch 18 oder 19, worin das vorgenannte Protein der Tumor Necrosis Factor (TNF-Alpha) ist.

**23.** Verfahren zur Identifizierung von proteolytischen Aktivitäten und/oder von Inibitoren dieser Aktivitäten, welches,

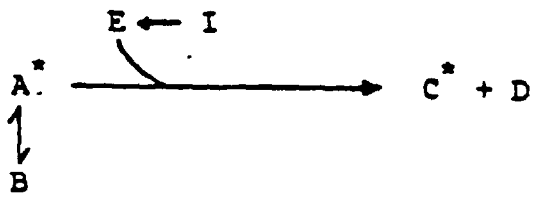


enthält, worin

E die vorgenannte proteolytische Aktivität darstellt, die die Konversion eines Substrats A in Fragmente C und D zu katalisieren imstande ist, wobei das vorgenannte Substrat A mindesten ein mit mindestens einer proteolytischen Angriffsstelle etikettiertes Proteinfragment enthält; I die vorgenannte inibitorische Aktivität gegenüber E darstellt; B einen Ligand darstellt, der das vorgenannte Substrat A, aber nicht die vorgenannten Fragmente C und D zu erkennen imstande ist, wobei B aus Molekülen besteht, die ihrerseist aus einem mindestens eine Aminosäure mit mindestens zwei Endaminofunktionen und einer linearen Aminosäuresequenz enthaltenden Aminosäurekern bestehen, wobei diese Sequenz hydropatisch komplementär zum vorgegenannte Proteinfragment ist und durch eine Karbamid-Kopelung mit je einer der vorgenannten Endaminofunktionen gebunden ist;
* gibt an, dass das vorgenannte Substrat etikettiert ist.

**24.** Verfahren zur Identifizierung der proteolitischen Aktivitäten und/oder der Inhibitoren dieser Aktivitäten nach Anspruch 23, worin die vorgenannten proteolitischen Aktivitäten oder deren Inhibitore in biologischen Flüssigkeiten oder in Gärungsbrühen oder in Zellextrakten vorhanden sind.

**25.** Verfahren zur Identifizierung der proteolitischen Aktivitäten und/oder der Inhibitoren dieser Aktivitäten nach je einem der vorhergehenden Ansprüche 23 oder 24, worin das vorgenannte Proteinfragment durch Radioisotope, oder Enzyme, oder Fluoreszenzverbindungen, oder Chromophore, oder Biotin etikettiert ist.

**26.** Verfahren zur Identifizierung der proteolitischen Aktivitäten und/oder der Inhibitoren dieser Aktivitäten nach Anspruch 25, worin das vorgenannte Proteinfragment durch Derivatization von Biotin etikettiert ist und durch Inkubation mit der Peroxidase, mit Streptavidin konjugiert ist.

**27.** Verfahren zur Identifizierung der proteolitischen Aktivitäten und/oder der Inhibitoren dieser Aktivitäten nach je einem der vorhergehenden Ansprüche 23 bis 26, worin auch Mittel zum Abscheiden des Protein-Ligand-Fragment-Komplexes von den nicht gebunden Prteinfragmenten und von den proteolyzierten Derivaten vorgesehen sind.

**28.** Verfahren zur Identifizierung der proteolitischen Aktivitäten und/oder der Inhibitoren dieser Aktivitäten nach Anspruch 27, worin die vorgenannten Abscheidemittel durch Immobilisierung des vorgenannte Ligands in der festen Phase und Abwaschen der vorgenannten Proteinfragmente und der vorgenannten proteolyzierten Derivative geschaffen werden.

**29.** Verfahren zur Identifizierung der proteolitischen Aktivitäten und/oder der Inhibitoren dieser Aktivitäten nach Anspruch 28, worin die vorgenannte feste Phase in Abteilunge geteilte Kunststoffplatten enthält.

**30.** Verfahren zur Identifizierung der proteolitischen Aktivitäten und/oder der Inhibitoren dieser Aktivitäten nach je einem der vorhergehenden Ansprüche 27 bis 29, worin ein Verfahrenschritt zur Bestimmung der vorgenannten etikettierten Proteinfragmente vorgesehen ist, die mit dem vorgenannten, auf einer fersten Phase immobilisierten Liganden gebunden sind.

**31.** Verfahren zur Identifizierung der proteolitischen Aktivitäten und/oder der Inhibitoren dieser Aktivitäten nach je einem der vorhergehenden Ansprüche 23 bis 30, worin das vorgenannte Substrat ein Fragment von Pro-Endothelin und das vorgenannte Ligand Aminosäuresequenze enthält, die zum vorgenannten Fragment von Pro-Endothelin hydropatisch komplementär sind.

**32.** Verfahren zur Identifizierung der proteolitischen Aktivitäten und/oder der Inhibitoren dieser Aktivitäten nach Anspruch 31, worin dass vorgenannte Fragment von Pro-Endothelin aus der Sequenz der Aminosäuren 16 bis 32

der Formel:

HLDIIWVNTPEHIVPYG

besteht und das vorgenannte Ligand das Molekül 8 Δ ET (16-29) ist.

**33.** Verfahren zur Identifizierung der proteolitischen Aktivitäten und/oder der Inhibitoren dieser Aktivitäten nach Anspruch 32, worin die vorgenannte proteolitische Angriffstelle sich zwischen den Aminosäuren 21 und 22 des vorgenannten Fragments 16-32 von Pro-Endothelin befindet, um so in Anwesenheit von proteolitischen Aktivitäten zwei Fragmente ET (16-21) und ET (22-32) zu erzeugen, die sich an das vorgenannte Moleküle 8 Δ ET zu binden nicht imstande sind.

## Revendications

**1.** Peptides non-linéaires complémentaires hydropeptiquement à des peptides ou portions des protéines ayants des séquences connues d'acides aminés, lesdits peptides étant caractérizés en ce qu'ils comprendent:

- un noyau d'acide aminé comprenant au moins un acide aminé avec au moins deux amino-fonctions terminales;
- une séquence linéaire d'acides aminés, qui est complémentaire à ladite séquence connue d'acides aminés et est liée par un carbamide-liaison à chacune desdites amino-fonctions terminales.

**2.** Peptides non-linéaires selon la revendication 1, dans lesquels ladite séquence hydropathiquement complementaire consiste d'un nombre des acides aminés entre 10 et 20.

**3.** Peptides non-linéaires selon une quelconque des revendications précédentes, dans lesquels ledit noyau d'acide amine a dans la position centrale la formule suivante

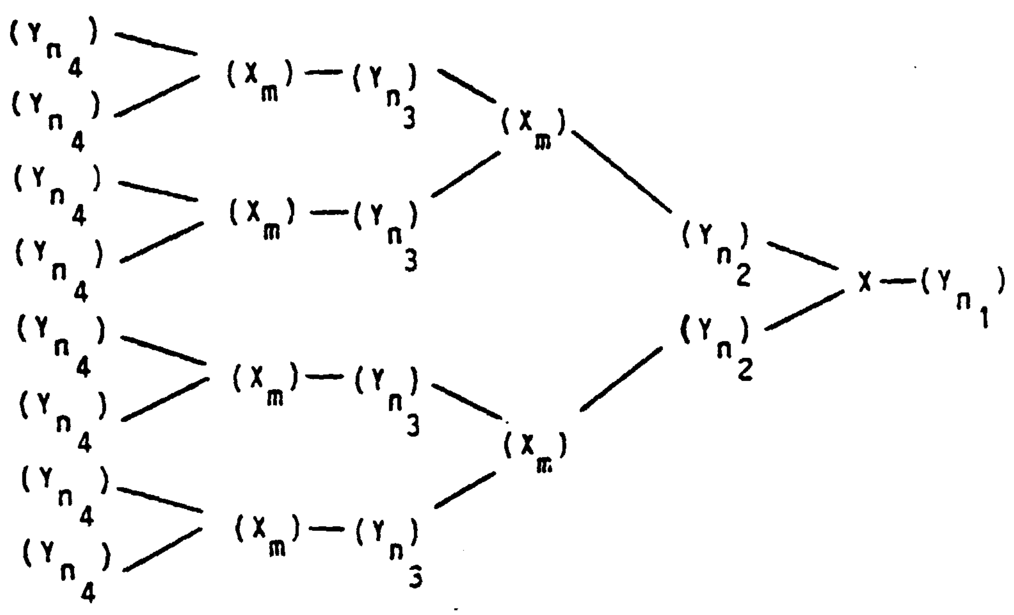

dans laquelle X est un acide aminé ayant au moins deux résidus amino-fonctionnels; Y est un acide aminé choisis du groupe de alanine, glycine ou arginine; m est un nombre compris entre 0 et 1; $n_1$, $n_2$, $n_3$, et $n_4$ sont des nombres entre 0 et 10 et dans lesquels les liaisons sont carbamideliaisons.

**4.** Peptides non-lineaires selon une quelconque des revendications précedentes, dans lesquels ledit acide aminé avec au moins deux amino-fonctions terminales (X) est lysine.

5. Peptides non-linéaires selon une quelconque des revendications précédentes 1-3, dans lesquels ledit acide aminé avec au moins deux amino-fonctions terminales (X) est ornitine.

6. Peptides non-lineaires selon une quelconque des revendications précedentes, dans lesquels ladite séquence à une portion de Big-Endothelin.

7. Peptides non-lineaires selon la revendication 6, dans lesquelles ladite séquence est hydropathiquement complémentaire à la portion de Big-Endothelin des acides aminés 16 à 20.

8. Peptides non-lineaires selon la revendication 7, dans lesquels ladite séquence a la formule suivante:

RKFLAGLRARRLKF.

9. Peptides non-linéaires selon une quelconque des revendications précédentes 1-5, dans lesquels ladite séquence des acides aminés est hydropathiquement complémentaire à une portion de TNF alpha.

10. Peptides non-lineaires selon la revendication 9, dans lesquels ladite séquence est hydropathiquement complémentaire à la portion 144-157 de TFN alpha.

11. Peptides non-linéaires selon la revendication 10, dans lesquels ladite séquence a la formule suivante:

DYLAGFKAHGKKYR.

12. Procédé de synthèse des peptides non-linéaires selon une quelconque des revendications précédentes, ledit procédé comprenant les phases de:

- immobilisation sur une phase solide du résidu d'un acide aminé;
- laisser former un carbamide liaison entre le amino-résidu dudit acide aminé et le carboxyl-résidu d'un acide aminé ayant au moins deux amino-fonctions terminales;
- addition à chacun desdites amino-fonctions terminales d'un acide aminé ayant lui même au moins deux amino-fonctions terminales;
- répétition de 0 à 2 fois de ce phase;
- synthèse des peptides linéaires ayants la liaison covalente à chacune desdites amino-fonctions terminales.

13. Procédé selon la revendication 12, dans lequel des acides aminés choisis du groupe de glycine, alanine ou arginine sont interposés de façon à agir comme entretoises entre les acides aminés ayant au moins deux amino-fonctions terminales.

14. Peptides non-lineaires selon la revendication 12, dans lequel ledits peptides sont hydropathiquement complémentaires à au moins une portion dedit Big-Endothelin.

15. Procédé selon la revendication 12, dans lequel lesdits peptides sont hydropathiquement complémentaires à au moins une portion dedit TNF alpha.

16. Procédé pour la préparation des colonnes chromatographiques d'affinité en utilisant des peptides non-linéaires selon les revendications précédentes 1 à 11, comprenant les phases suivantes

- immobilisation du support derivé sur une colonne chromatographique et mise en équilibre de ladite colonne avec un tampon salin;
- injection dans la colonne d'une quantité d'un composé brut contenant la protéine ou le peptide à épurer, ladite quantité étant compatible avec la force de separation de la colonne;
- lavage de la colonne avec un tampon d'élution jusq'à élimination des matières étrangers et l'élution de ladite protéine ou dudit peptide liés à la colonne au moyen d'un échange d'éluent de façon à contrarier la liaison.

17. Procédé selon la revendication 16, dans lequel ladite protéine à épurer est Big-Endothelin.

**18.** Procédé selon la revendication 16, dans lequel ladite protéine à épurer est le Tumor Necrosis Factor (TNF alpha).

**19.** Procédé pour la détermination des peptides ou protéines d'origine naturel ou d'origine recombinante dans échantillons, ledit procédé comprenant:

- le traitement d'un récipient réparti en secteurs avec des peptides non-linéaires selon les revendications 1 à 11;
- l'etiquetage d'une quote-part des peptides ou des protéines à déterminer;
- l'incubation desdits peptides ou protéines étiquetés dans ledit récipient;
- l'incubation de l'échantillon contenant ledit peptide ou ladite protéine à déterminer dans ledit récipient;
- le relèvement de la presence dudit peptide au de ladite protéine ou moyen des déterminations différentieles.

**20.** Procédé selon la revendication 19, dans lequel ladite étiquetage est réalisée avec des radioisotopes ou par des réactions enzymiques ou avec un groupe fluorescent ou chromophorique.

**21.** Procédé selon les revendications 18 ou 19, dans lequel ladite protéine à déterminer est Big-Endothelin.

**22.** Procédé selon les revendications 18 ou 19, dans lequel ladite protéine est le Tumor Necrosis Factor (TNF alpha).

**23.** Procédé pour l'identification des activités protéolytique et/ou des inhibiteurs de telles activités, ledit procede comprenant

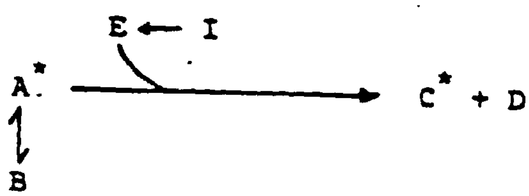


dans lequel

E raprésente ladite activité protéolytique, qui est susceptible de catalyser la conversion d'un substrat A en des fragments C et D, ledit substrat A comprenant au moins un fragment de protéine étiqueté avec au moins un lieu d'attaque protéolytique;
I raprésente ladite activité d'inhibition à l'égard de E
B raprésente un liant qui est susceptible de reconnaître ledit substrat mai pas ledits fragments C and D; B consistant des molecules formés par des noyaux comprenants au moins un acide aminé ayant au moins deux functions terminales et une séquence linéaire des acides aminés, qui est hydropathiquement complémentaire audit fragment de protéine et est liée par une carbamide liaison à chacune des amino fonctions terminales,
* indique que ledit substrat A est étiqueté.

**24.** Procédé pour l'identification des activités protéolytique et/ou des inhibiteurs de telles activités selon la revendication 23, dans lequel lesdites activités protéolytiques et/ou activité qui inhibitent les mêmes sont présentes dans les fluides biologiques ou dans les bouillons de fermentation ou dans les extraits des celles.

**25.** Procédé pour l'identification des activités protéolytique et/ou des inhibiteurs de telles activités selon une quelconque des revendications précédentes, dans lequel ledit fragment de protéin est étiqueté par les radio-isotopes ou enzymes ou composés fluorescents ou chromophores ou biotine.

**26.** Procédé pour l'identification des activités protéolytique et/ou des inhibiteurs de telles activités selon la revendication 25, dans lequel ledit fragment de protéine est étiqueté par derivation de la biotine et est évidencé par incubation avec la streptavidine conjuguée à la peroxidase.

**27.** Procédé pour l'identification des activités protéolytique et/ou des inhibiteurs de telles activités selon une quelconque des revendications précédentes 23-26, ledit procédé comprenant en outre des moyens pour la séparation du fragment complexe de liant-protéine des fragments de protéine qui ne sont pas liés et des dérivés protèolyzés.

**28.** Procédé pour l'identification des activités protéolytique et/ou des inhibiteurs de telles activités selon la revendication 27, dans lequel lesdit moyens de séparation sont réalisés par immobilisation dedit liant dans la phase solide et par lavage desdit fragment de protéine non-liés et desdits dérivés protéolysés.

**29.** Procédé pour l'identification des activités protéolytique et/ou des inhibiteurs de telles activités selon la revendication 28, dans lequel ladite phase solide comprend des plaques de matière plastique répartie en sécteurs.

**30.** Procédé pour l'identification des activités protéolytique et/ou des inhibiteurs de telles activités selon une quelconque des revendications précédentes 27 à 29, comprenant la phase de détermination desdits fragments de protéine étiquetés, qui sont liés audit liant immobilisé sur une phase solide.

**31.** Procédé pour l'identification des activités protéolytique et/ou des inhibiteurs de telles activités selon les revendications 23 à 30, dans lequel ledit substrat comprend un fragment de pro-endothéline et ledit liant comprend des séquences des acides aminés, qui sont hydropathiquement complementaires audit fragment de pro-endothéline.

**32.** Procédé pour l'identification des activités protéolytique et/ou des inhibiteurs de telles activités selon la revendication 31, dans lequel ledit fragment de pro-endothéline est formé de la séquence des acides aminés 16 à 32, ayants la formule:

HLDIIWVNTPEHIVPYG

et ledit liant est la molecule 8 Δ ET (16-29).

**33.** Procédé pour l'identification des activités protéolytique et/ou des inhibiteurs de telles activités selon la revendication 32, dans lequel ledit lieu d'attaque protéolytique est entre les acides aminés 21 et 22 dudit fragment 16-32 de pro-endothéline, de façon à générer en présence des activités protéolytiques deux fragments ET (16-21) et ET (22-32) qui ne sont pas susceptibles de lier ladite molécule 8 Δ ET (16-29).

FIG. 1A

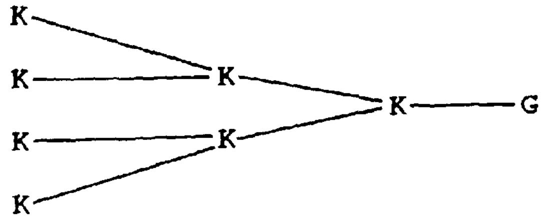


FIG. 1B

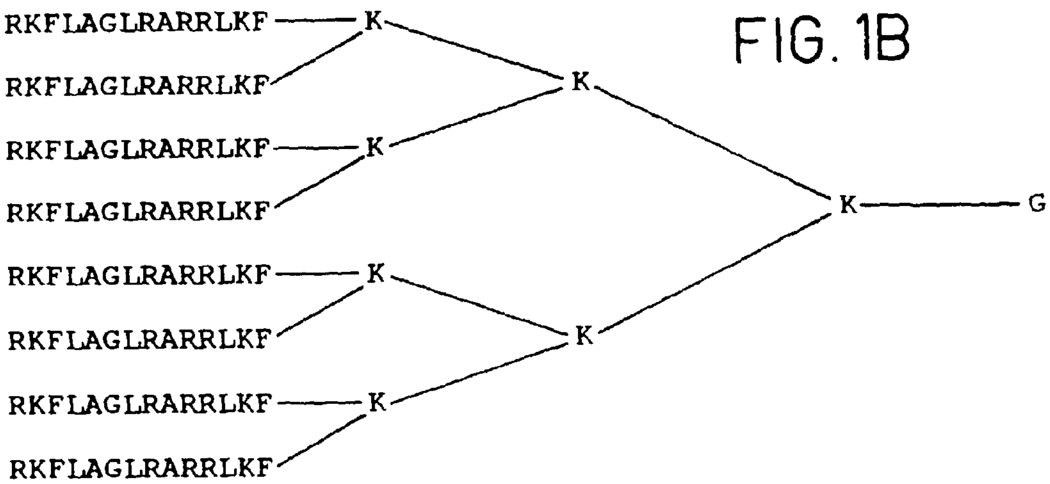


FIG. 1C

HLDIIWVNTPEHIV

FIG. 1D

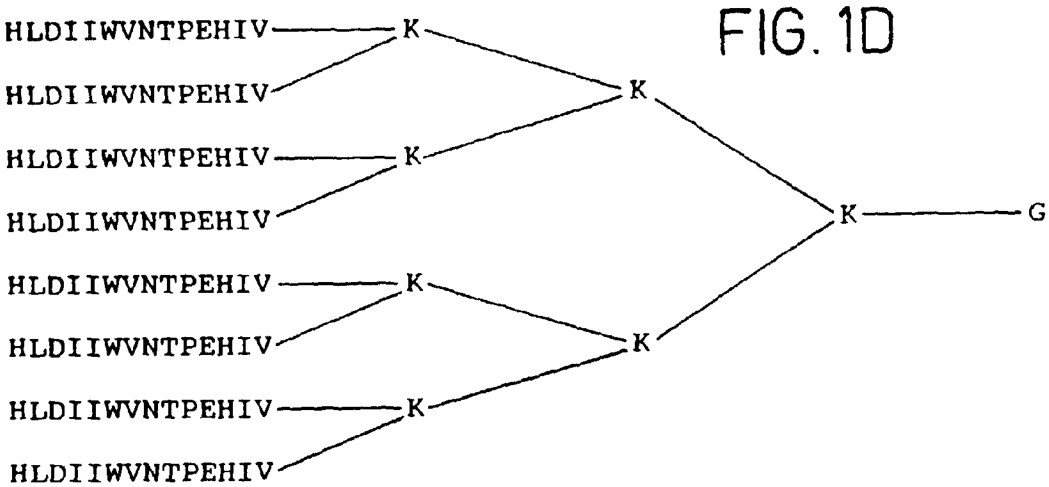

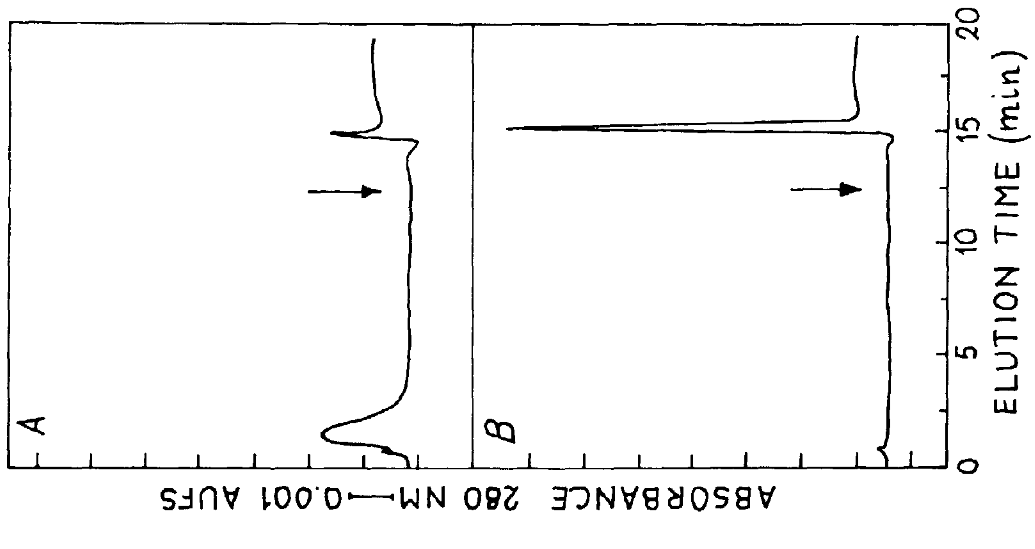
A
B
ABSORBANCE 280 NM — 0.001 AUFS
ELUTION TIME (min)
0
5
10
15
20

FIG. 2

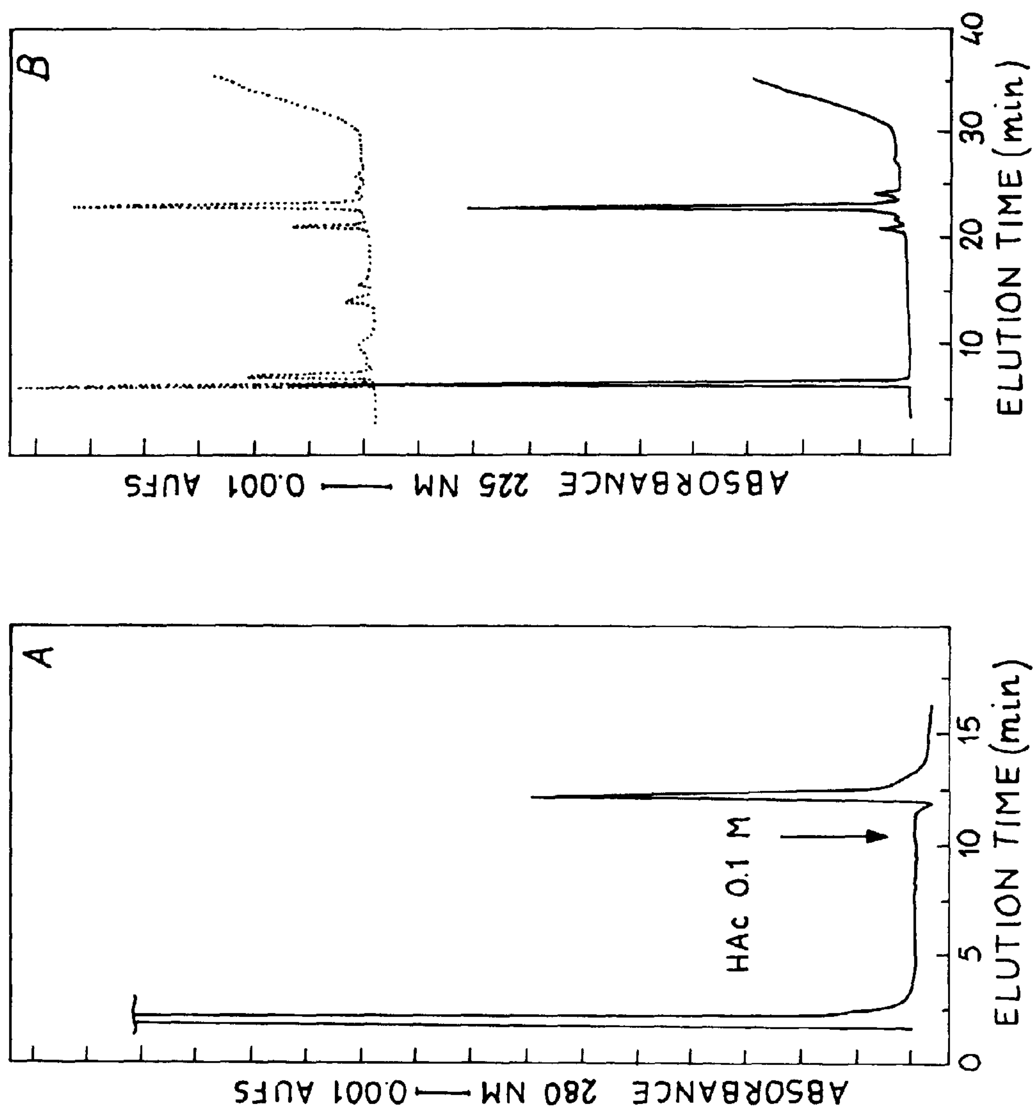
A
HAc 0.1 M
ABSORBANCE 280 NM — 0.001 AUFS
ELUTION TIME (min)
0
5
10
15
B
ABSORBANCE 225 NM — 0.001 AUFS
ELUTION TIME (min)
10
20
30
40

FIG. 3

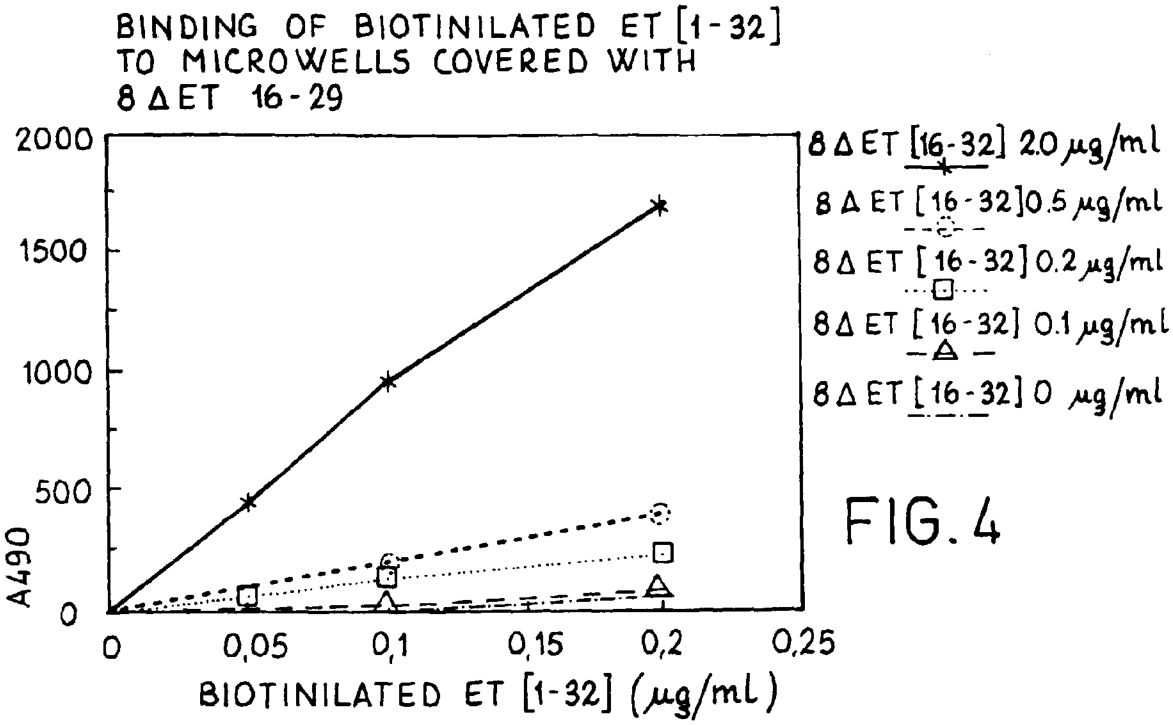
BINDING OF BIOTINILATED ET [1-32] TO MICROWELLS COVERED WITH 8 Δ ET 16-29
2000
1500
1000
500
0
A490
0
0,05
0,1
0,15
0,2
0,25
BIOTINILATED ET [1-32] (μg/ml)
8 Δ ET [16-32] 2.0 μg/ml
8 Δ ET [16-32] 0.5 μg/ml
8 Δ ET [16-32] 0.2 μg/ml
8 Δ ET [16-32] 0.1 μg/ml
8 Δ ET [16-32] 0 μg/ml

FIG. 4

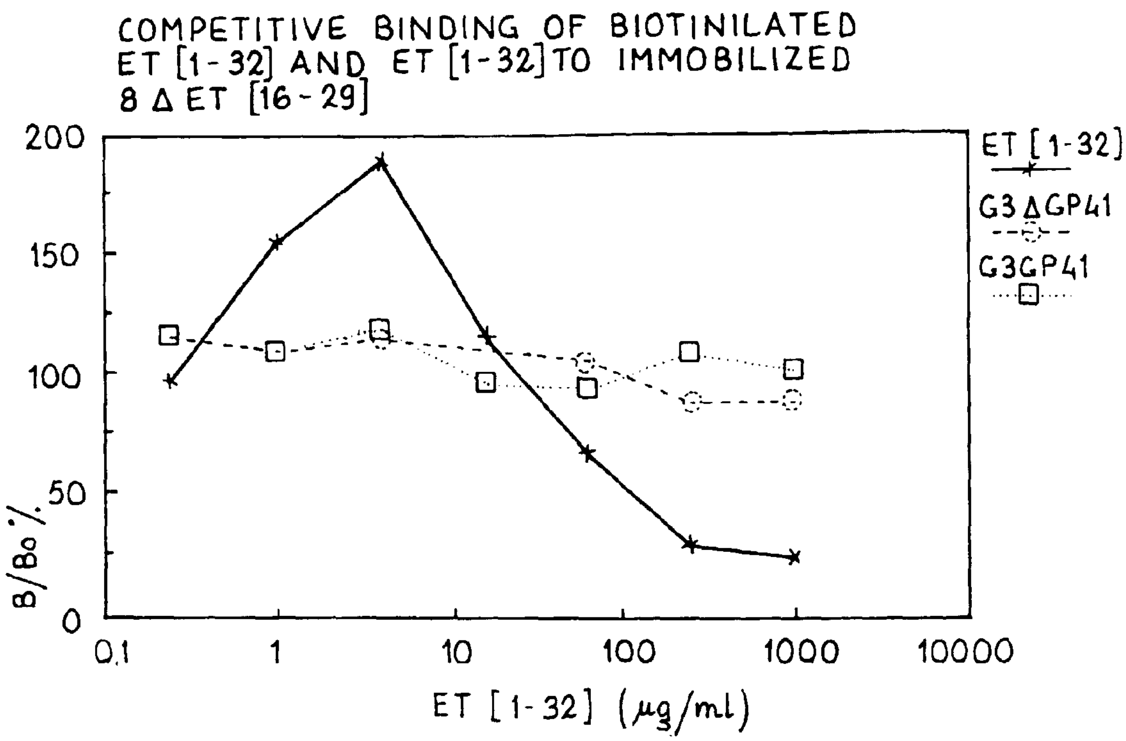
COMPETITIVE BINDING OF BIOTINILATED ET [1-32] AND ET [1-32] TO IMMOBILIZED 8 Δ ET [16-29]
200
150
100
50
0
B/Bo %
0,1
1
10
100
1000
10000
ET [1-32] (μg/ml)
ET [1-32]
G3ΔGP41
G3GP41

FIG. 5

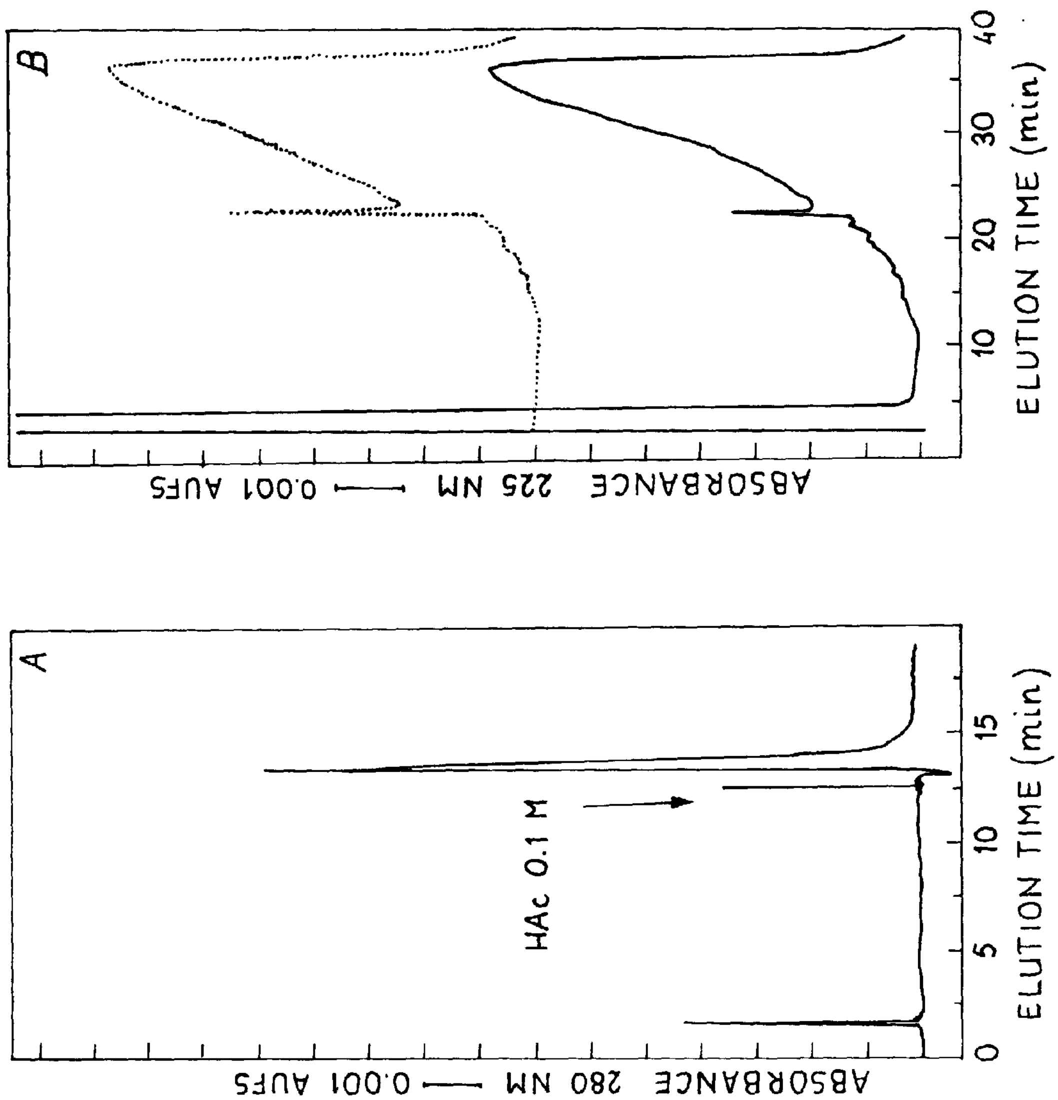
A
B
ABSORBANCE 280 NM —— 0.001 AUFS
ABSORBANCE 225 NM —— 0.001 AUFS
HAc 0.1 M
ELUTION TIME (min)
0
5
10
15
20
30
40

FIG. 8

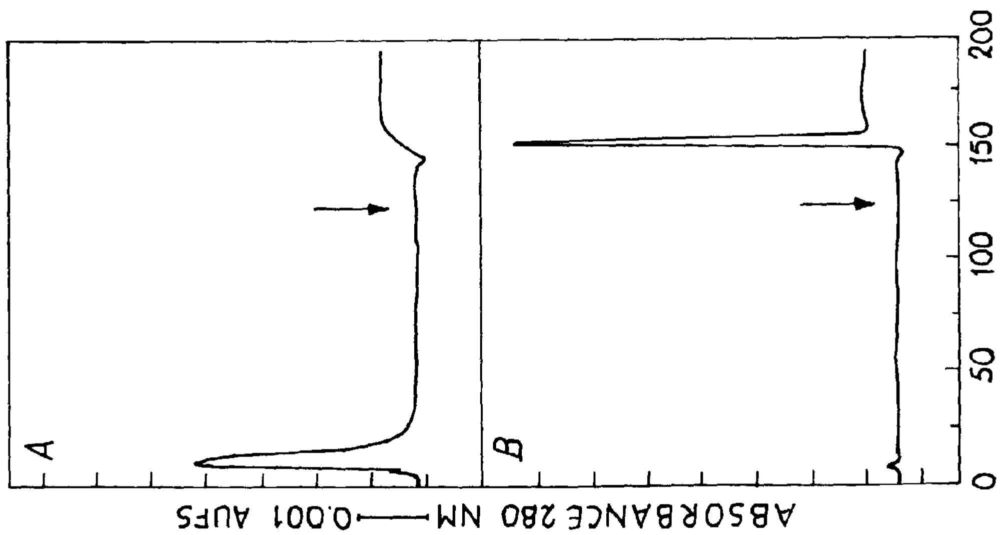
A
B
ABSORBANCE 280 NM —— 0.001 AUFS
ELUTION TIME (min)
0
50
100
150
200

FIG. 6

FIG. 7a

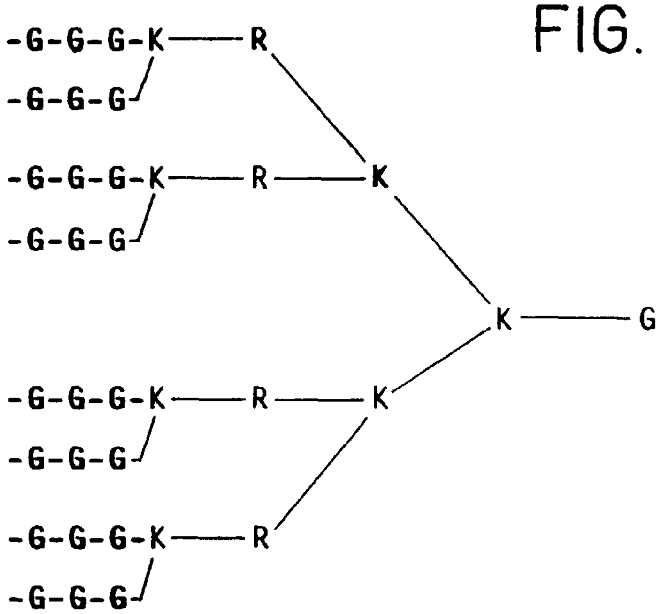
-G-G-G-K——R
-G-G-G
-G-G-G-K——R——K
-G-G-G
K——G
-G-G-G-K——R——K
-G-G-G
-G-G-G-K——R
-G-G-G

FIG. 7b

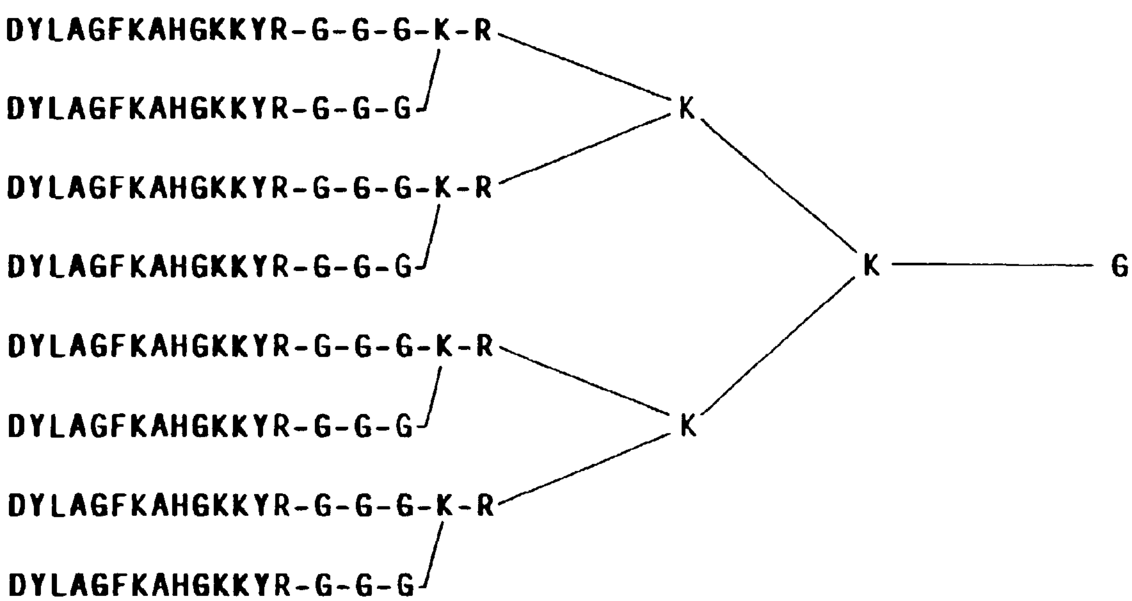
DYLAGFKAHGKKYR-G-G-G-K-R
DYLAGFKAHGKKYR-G-G-G
K
DYLAGFKAHGKKYR-G-G-G-K-R
DYLAGFKAHGKKYR-G-G-G
K——G
DYLAGFKAHGKKYR-G-G-G-K-R
DYLAGFKAHGKKYR-G-G-G
K
DYLAGFKAHGKKYR-G-G-G-K-R
DYLAGFKAHGKKYR-G-G-G

# FIG. 9

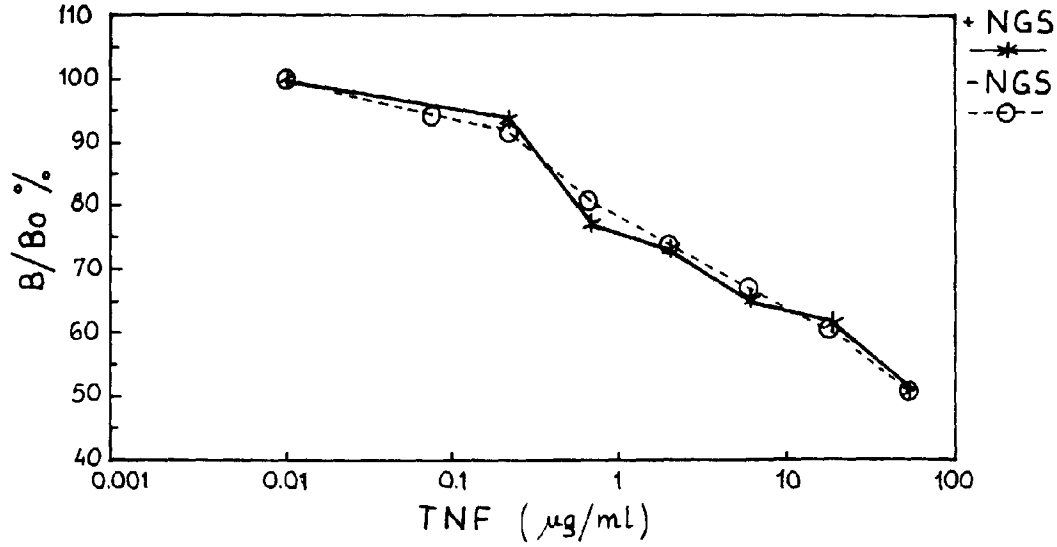


PROTEOLYTIC DEGRADATION KINETICS

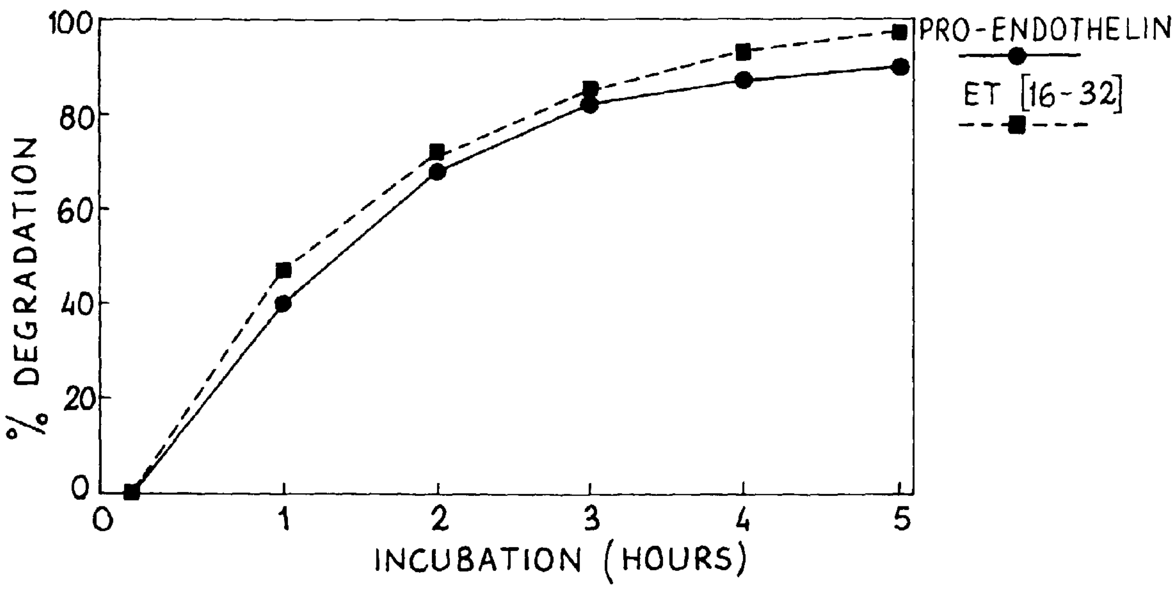


PRO-ET [50 µg/ml]
ET[16-32] [50 µg/ml]

# FIG. 10

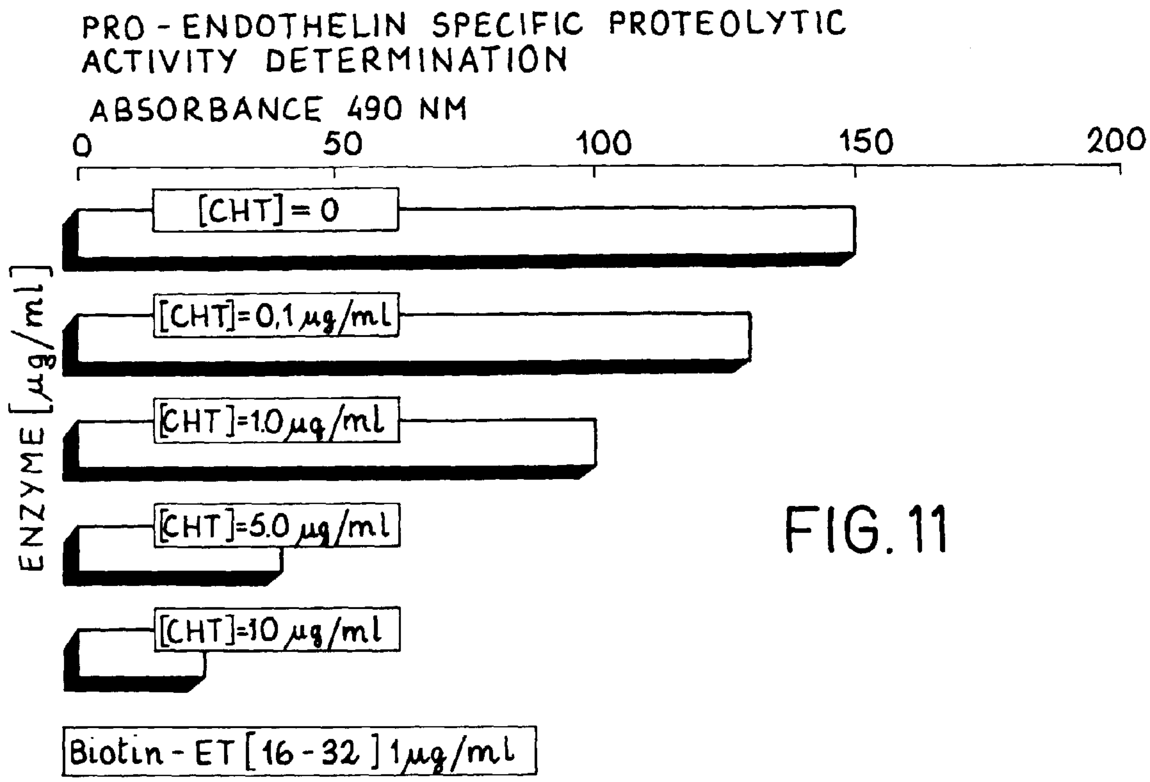
PRO - ENDOTHELIN SPECIFIC PROTEOLYTIC ACTIVITY DETERMINATION
ABSORBANCE 490 NM
0
50
100
150
200
[CHT] = 0
[CHT]=0,1 μg/ml
[CHT]=1.0 μg/ml
[CHT]=5.0 μg/ml
[CHT]=10 μg/ml
ENZYME [μg/ml]
Biotin - ET [16 - 32] 1μg/ml

FIG. 11

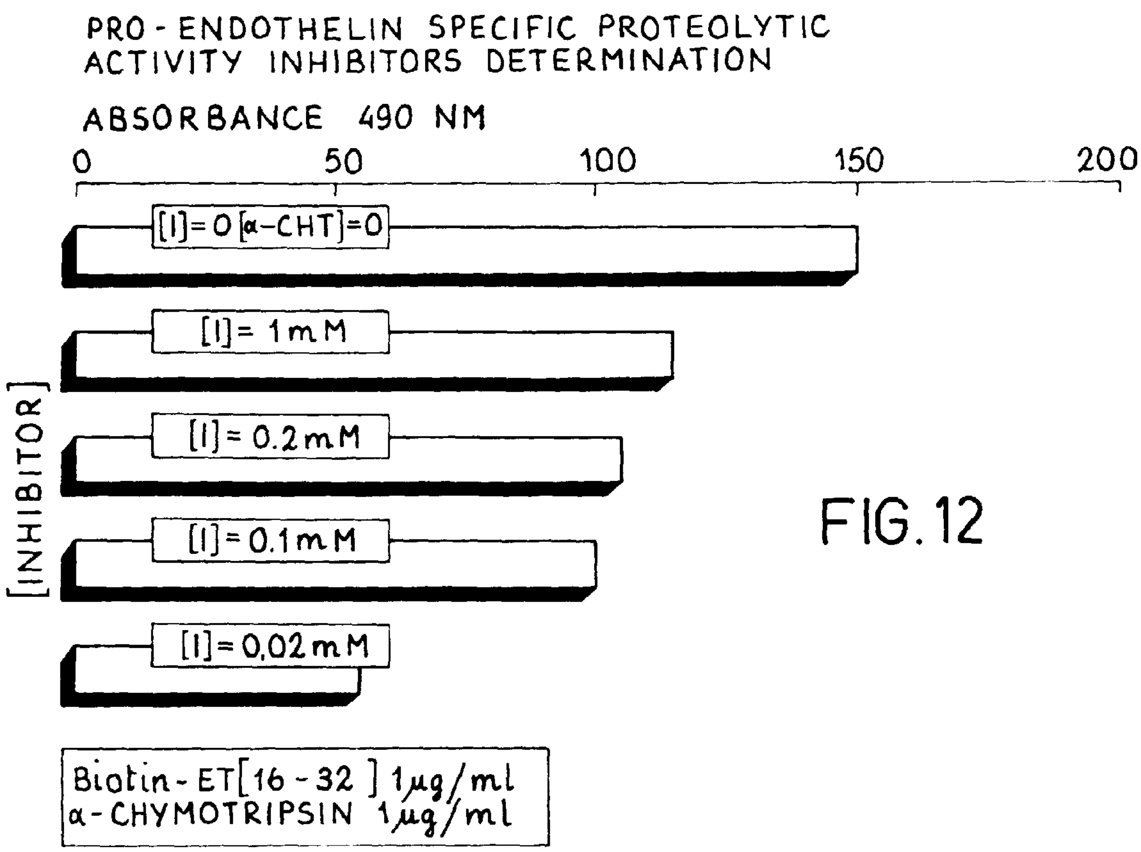
PRO - ENDOTHELIN SPECIFIC PROTEOLYTIC ACTIVITY INHIBITORS DETERMINATION
ABSORBANCE 490 NM
0
50
100
150
200
[I]= 0 [α-CHT]=0
[I]= 1mM
[I]= 0.2mM
[I]= 0.1mM
[I]= 0,02mM
[INHIBITOR]
Biotin - ET[16 - 32] 1μg/ml
α - CHYMOTRIPSIN 1μg/ml

FIG. 12